# FASCICULE DE BREVET EUROPEEN

(11) **EP 1 226 133 B1**
(45) Date de publication et mention de la délivrance du brevet: **07.04.2004**
(21) Numéro de dépôt: 00969660.0
(22) Date de dépôt: 20.10.2000
(51) Int. Cl.: C07D 307/33, A61K 31/365, A61P 25/00, A61P 25/08, A61P 25/22

(54) **3-AMINO-2,2-DI-C-ALKYL-1,4-BUTYROLACTONES ET 1,4-THIOBUTYROLACTONES N-SUBSTITUEES UTILES COMME STIMULANT DE L'ACTIVITE DE L'ACIDE $g(g)-AMINOBUTYRIQUE ET DANS LE TRAITEMENT DES TROUBLES NERVEUX ET LEUR PROCEDE DE PREPARATION**
3-AMINO-2,2-DI-C-ALKYL-1,4-BUTYROLACTONE UND N-SUBSTITUIERTE 1,4-THIOBUTYROLACTONE DIE ZUR STIMULATION DER GAMMA-AMINOBUTANSÄUREAKTIVITÄT UND ZUR BEHANDLUNG VON NERVENERKRANKUNGEN VERWENDUNG FINDEN, SOWIE VERFAHREN ZU IHRER HERSTELLUNG
N-SUBSTITUTED 3-AMINO-2,2-DI-C-ALKYL-1,4-BUTYROLACTONES AND 1,4-THIOBUTYROLACTONES FOR USE AS PROMOTER OF $g(g)-AMINOBUTYRIC ACID ACTIVITY AND FOR TREATING NERVOUS DISORDERS AND PREPARATION METHOD

(30) Priorité: 22.10.1999 FR 9913233
(43) Date de publication de la demande: 31.07.2002
(73) Titulaire: CENTRE NATIONAL DE LA RECHERCHE SCIENTIFIQUE (CNRS), 75794 Paris Cedex 16 (FR); Innovationsagentur GmbH bu Technology Marketing Austria (TECMA), 1020 Vienne (AT); Université de Bern, 3012 Bern (CH)
(72) Inventeur: DODD, Robert, F-75009 Paris (FR); EL HADRI, Ahmed, F-91240 Saint-Michel-sur-Orge (FR); POTIER, Pierre, Jean-Paul, F-75007 Paris (FR); SIEGHART, Werner, A-1190 Vienne (AT); JURSKY, Frantisek, Bratislava (SK); FURTMULLER, Roman, A-3143 Pyra (AT); SIGEL, Erwin, CH-3047 Bremgarten (CH); THOMET, Urs, CH-4114 Hofstetten (CH)
(74) Mandataire: Ahner, Francis
(86) Numéro de dépôt international: PCT/FR2000/002928
(87) Numéro de publication internationale: WO 2001/029017

(56) Documents cités:
- EP-A- 0 151 964
- US-A- 3 968 233
- D.J. CANNEY ET AL.: "Synthesis and Structure-Activity Studies of Alkyl-Substituted gamma-Butyrolactones and gamma-Thiobutyrolactones: Ligands for the Picrotoxin Receptor" JOURNAL OF MEDICINAL CHEMISTRY, vol. 34, 1991, pages 1460-1467, XP002146077 WASHINGTON US cité dans la demande
- JEFFORD C W ET AL: "185.A PRACTICAL SYNTHESIS OF (2S,3R)-3-AMINO-2-METHYLPENTANOIC ACIDFROM L-ASPARTIC ACID" HELVETICA CHIMICA ACTA,CH,VERLAG HELVETICA CHIMICA ACTA. BASEL, vol. 77, no. 8, 1994, pages 2142-2146, XP002070485 ISSN: 0018-019X

## Description

La présente invention concerne des 3-amino-2,2-di-C-alkyl-1,4-butyrolactones et 1,4-thiobutyrolactones N-substituées, leur préparation, les compositions pharmaceutiques les comprenant et leur utilisation comme stimulant de l'activité de l'acide γ-aminobutyrique et comme médicament destiné de préférence au traitement des troubles nerveux.

### LE RECEPTEUR GABA-A

L'acide γ-aminobutyrique (ou GABA (1)) est le neurotransmetteur inhibiteur le plus important du système nerveux central. Il agit au niveau de trois classes distinctes de récepteurs dénommés récepteurs GABA-A, GABA-B et GABA-C. Le récepteur GABA-A, dont la séquence d'acides aminés a été déterminée par des techniques de clonage, est une structure pentamérique composée de sous-unités α, β, γ, δ et/ou ρ. Jusqu'à présent, 6 sous-unités α, 3 sous-unités β, 3 sous-unités γ, 1 sous-unité δ et 2 sous-unités ρ ont été identifiées et séquencées. Cinq de ces sous-unités (par exemple 2α₁ 2β₂ γ₂) se rassemblent pour former un canal perméable aux ions chlorure. En se liant à ce récepteur GABA-A, le GABA accroît la perméabilité du canal aux ions chlorure, inhibant ainsi la transmission neuronale. Au vu du grand nombre de permutations possibles des diverses sous-unités, une très grande hétérogénéité du récepteur GABA-A est observée dans le cerveau des mammifères et différentes structures du cerveau montrent généralement une prépondérance pour certaines combinaisons de sous-unités.

La recherche de ligands sélectifs de l'une de ces différentes sous-classes de récepteurs GABA-A est un objectif majeur de la recherche clinique médicale dans ce domaine.

En dehors du GABA, on connaît un grand nombre de différentes classes de composés se liant au récepteur GABA-A. Certains produits, tels que le muscimol et l'isoguvacine, se lient directement au même site que le GABA sur le récepteur GABA-A et stimulent le récepteur de la même façon que le GABA lui-même. A l'opposé de ces agonistes, certaines substances, comme la bicuculline (2), inhibent de façon compétitive l'action du GABA. De tels antagonistes du récepteur du GABA montrent des propriétés convulsivantes *in vivo* (P. Krogsgaard-Larsen, B. Frolund, F.S. Jorgensen, A. Schousboe, J. Med. Chem., 1994, 37, 2489).

L'action inhibitrice du GABA peut être modulée par des composés qui interagissent avec une variété de sites allostériques sur le récepteur GABA-A distincts du site de reconnaissance du GABA. Une des classes les plus connues de modulateurs allostériques du récepteur GABA-A est celle des benzodiazépines (par exemple le diazépam (3)). En se liant ainsi à leur propre site de reconnaissance sur le récepteur GABA-A (le récepteur des benzodiazépines ou BZR), ces composés améliorent l'action du GABA en augmentant la fréquence d'ouverture du canal chlorure (RE. Study, J.L. Barker, Proc. Natl. Acad. Sci. USA, 1981, 78, 7180). Il en résulte les activités anticonvulsivante, anxiolytique, sédative-hypnotique et myorelaxante de ces produits largement utilisés en clinique. D'autres classes de composés structurellement non apparentées aux benzodiazépines, telles que les triazolopyridazines (par exemple Cl 218872 (**4**)), les imidazopyridines (par exemple le zolpidem (**5**)), les cyclopyrrolones (par exemple le zopicolone (**6**)) et les β-carbolines (par exemple le β-CCM (7)), peuvent également se lier aux récepteurs des benzodiazépines. Dans le cas de ces derniers, certains dérivés inhibent, plutôt qu'augmentent, l'action neuro-inhibitrice du GABA (R.L. Macdonald, R.E. Twyman in « Ion Channels » ed. by T. Narahashi, Vol. 3, pp. 315-343, Plenum Press, New York, 1992). Dans ce cas, les composés, généralement convulsivant, sont appelés agonistes inverses (ou modulateurs allostériques négatifs) du BZR, pour les distinguer des agonistes (ou modulateurs allostériques positifs) du BZR utiles thérapeutiquement. Certains de ces produits font preuve d'une sélectivité au niveau des diverses sous-classes de récepteurs GABA-A/benzodiazépines. Ainsi le zolpidem, utilisé cliniquement comme hypnotique, est sélectif pour la sous-classe de récepteurs des benzodiazépines que l'on trouve de façon prépondérante dans le cervelet (récepteurs BZ1) (S. Arbilla, H. Depoortere, P. George, S.Z. Langer, Naunyn-Schmiedeberg's Arch. Pharmacol., 1985, 330, 248). Cette sélectivité se traduit soit par un spectre d'activité plus étroit (par exemple, anxiolyse sans effet hypnotique) soit par une diminution des effets indésirables de ce type de produit (accoutumance, dépendance, amnésie ...).

D'autres sites existent sur le récepteur GABA-A qui permettent également, en fonction de sa liaison avec une molécule appropriée, de moduler l'activité du GABA. Parmi ces sites, citons ceux pour les neurostéroïdes (par exemple la 3α-OH-5α-pregnane-20-one), les barbituriques (par exemple le pentobarbital), les anesthésiants (par exemple le propofol), les convulsivants de cage t-butylbicyclophosphorothionate (par exemple le TBPS, **8**) qui se lient au site de la picrotoxine du récepteur GABA-A (W. Sieghart, Pharmacol. Rev., 1995, 47, 181 et C.R. Gardner, W.R. Tully, C.J.R. Hedgecock, Prog. Neurobiol., 1993, 40, 1). D'autres sites de liaison, moins bien caractérisés mais apparemment distincts, sont ceux du loreclézole et des γ-butyrolactones. De tels composés modulent aussi positivement l'action du GABA et cet effet est traduit en une action in vivo anticonvulsivante et/ou anxiolytique.

Récemment, il a été démontré que les γ-butyrolactones gem-dialkylés (**9a**) et les γ-thiobutyrolactones gem-dialkylés (**9b**) peuvent soit diminuer soit augmenter l'action du GABA selon la position et la taille de leurs substituants alkyles (K.D. Holland, M.G. Bouley, D.F. Covey, J.A. Ferrendelli, Brain Res., 1993, 615, 170). Ces composés inhibent allostériquement la liaison du [S³⁵]TBPS sur les membranes du cerveau du rat, mais ne déplacent pas le [H³]-flunitrazépam de son site de liaison, n'augmentant pas non plus la liaison des benzodiazépines ou du muscimol. Ceci suggère que les composés de type 9 peuvent agir sur un site différent de ceux déjà caractérisés sur le complexe récepteur GABA-A.

Finalement, le loreclézole (10) est un nouveau composé démontrant à la fois une activité anticonvulsivante et une activité anxiolytique dans des modèles animaux variés (A. Wauquier et al., Drug Dev. Res., 1990, 19, 375 et G.R. Dawson, R. Curnow, P. Bayley, A. Rambridge, M.D. Tricklebank, Eur. J. Pharmacol., 1994, 252, 325). Ces composés n'ont qu'une affinité négligeable pour les sites de reconnaissance des benzodiazépines. Bien que l'interaction directe du loreclézole avec les récepteurs GABA-A ait été démontrée dans les études de récepteur recombinant, la relation entre les sites de liaison du loreclézole et les autres sites de liaison allostérique de ce récepteur n'est pas claire à ce jour. Récemment, il a été démontré que l'affinité du loreclézole pour les récepteurs contenant les sous-unités β₂ ou β₃ était 300 fois plus grande que celle pour les récepteurs contenant la sous-unité β₁ (P.B. Wingrove, K.A. Wafford, C. Bain, P.J. Whiting, Proc. Natl. Acad. Sci. USA, 1994, 91, 4569). Cette sélectivité peut expliquer l'absence d'effets sédatifs du loreclézole et suggère que les composés interagissant avec le site de liaison du loreclézole sur le récepteur GABA-A peuvent avoir des applications thérapeutiques importantes.

Il est ainsi clair qu'un grand nombre de sites modulatoires allostériques, qui peuvent augmenter l'action du GABA et ainsi démontrer une efficacité thérapeutique dans une large gamme de désordres du système nerveux central, existent sur le récepteur GABA-A. Il peut ainsi être raisonnablement conclu que de nouvelles structures chimiques peuvent découvrir d'autres sites modulatoires allostériques à ce jour non caractérisés sur le récepteur GABA-A ou se lier à des sites connus avec de plus grandes affinités ou de plus grandes sélectivités. De tels composés peuvent, comme résultat, démontrer une activité puissante et/ou hautement spécifique de même que de plus faibles effets secondaires indésirables dans le traitement de tels désordres.

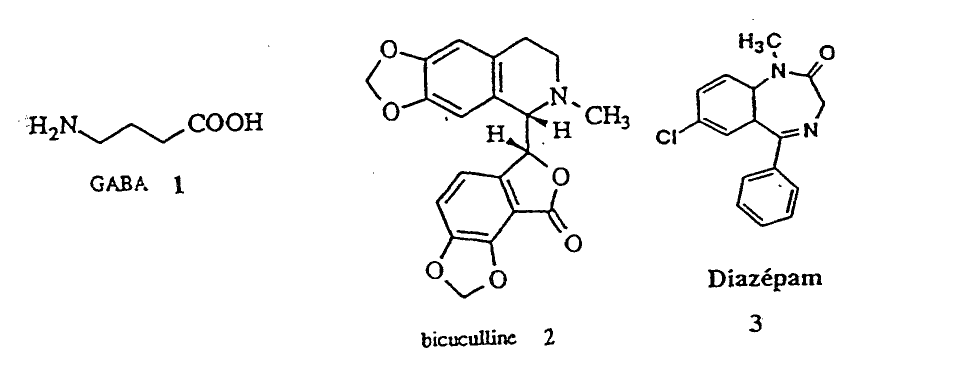

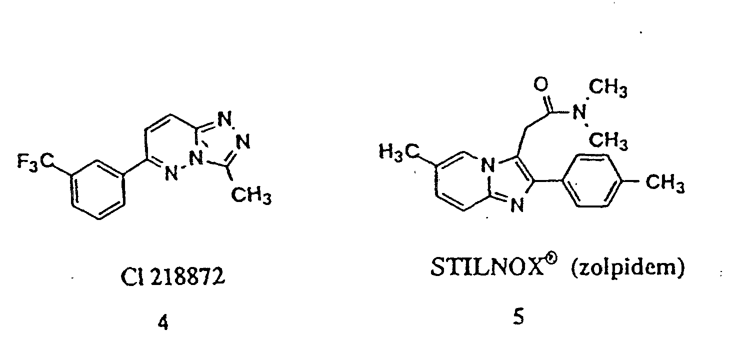

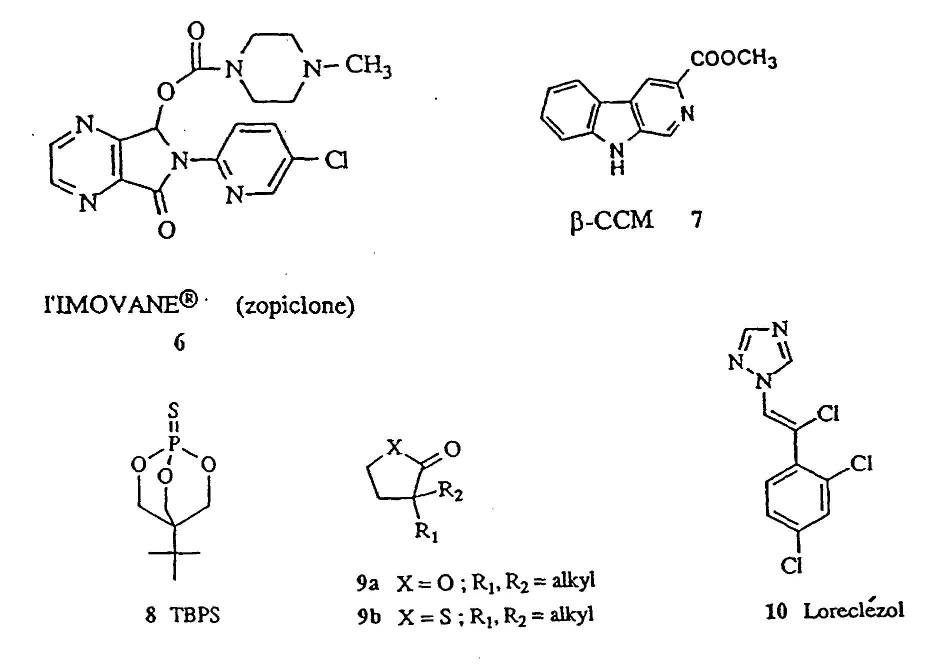

Covey et al ont été les premiers à signaler les propriétés convulsivantes et anticonvulsivantes des gem-dialkyl-1,4-butyrolactones (W.E. Klunk, A.C. McKeon, D.F. Covey, J.A. Ferrendelli, Science, 1982, 217, 1040). Ils ont trouvé qu'alors que les substitutions dialkyles à la position β (c'est-à-dire C-3) de la butyrolactone (par exemple le composé **54**) produit des effets convulsivants dans les souris, des substituants similaires à la position α (c'est-à-dire C-2) (par exemple le composé **55**) conduisent à des composés ayant des propriétés anticonvulsivantes dans les souris, empêchant les attaques induites par le pentylènetétrazole et la picrotoxine. Des études de structure-fonction sur le composé **55** indiquaient que l'activité anticonvulsivante était maintenue tant que les substituants alkyles contenaient 4 atomes de carbone ou moins. Si plus d'atomes de carbone étaient présents, des effets convulsivants étaient observés. Ces auteurs ont aussi étudié les α-alkyl substitués-1,4-thiobutyrolactones (par exemple **48**, α-EMTBL) (D.F. Covey et al., J. Med. Chem., 1991, 34, 1460), les cyclopentanones **56** et lactames (par exemple **57**). De même, des activités anticonvulsivantes ont été observées dans ces trois séries de composés avec des substituants alkyles à courte chaîne.

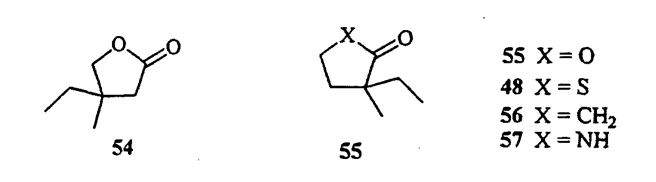

Le tableau 5 montre des données in vitro pour les exemples les plus actifs de ces composés dans leur habilité à déplacer le S³⁵-TBPS (indiquant une affinité avec le site de liaison de la picrotoxine du récepteur GABA-A) ainsi que leur capacité à stimuler des courants produits par le GABA dans des neurones cultivés.

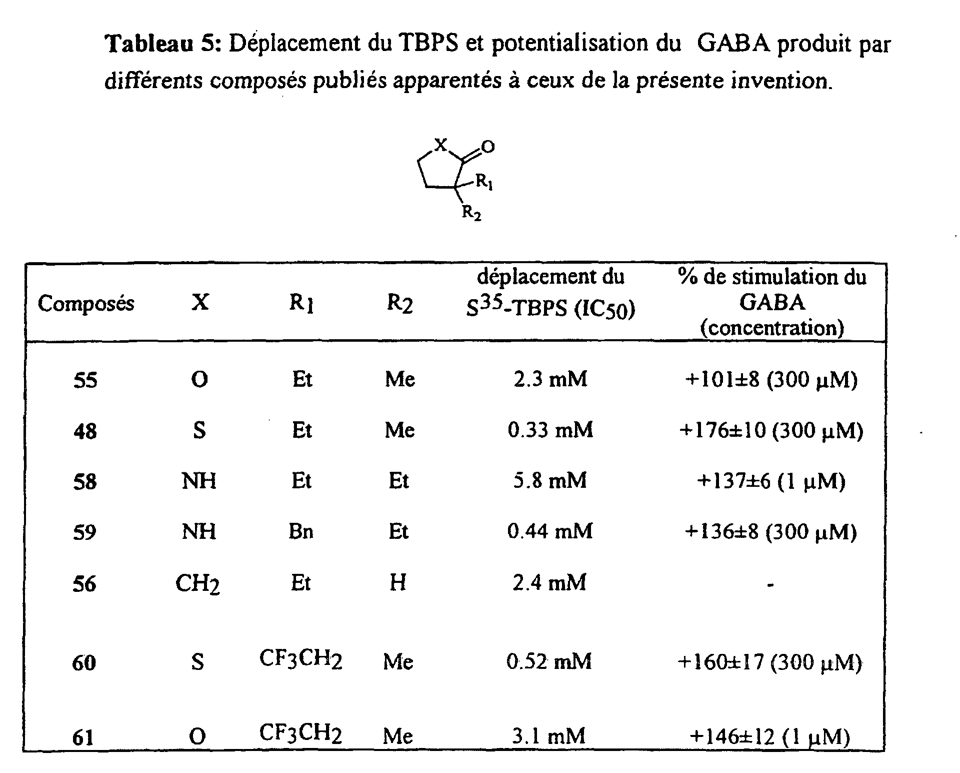

Les composés les plus actifs de l'invention (**30, 31, 34, 35**) sont généralement plus puissants à déplacer le TBPS (voir tableaux 2a et 2b) que les composés publiés montrés dans le tableau 5. De façon plus importante, les composés actifs de l'invention sont plus puissants à stimuler les courants produits par le GABA. Ainsi, alors que le pourcentage de stimulation des courants du GABA par des concentrations relativement haute (0,3-1 mM) des composés publiés étaient invariablement moins de +200%, les composés de la présente invention stimulent la réponse du GABA jusqu'à +700% et ceci à de plus basses concentrations (100 µM).

L'observation qu'il y a très peu de corrélation entre les puissances de déplacement du TBPS et la potentialisation du GABA (de même qu'avec la puissance anticonvulsivante) a conduit Covey et al à suggérer que les composés de cette famille (en particulier, l'α-EMTBL, **48,** sur lequel on a conduit le plus grand nombre d'études) agissent partiellement par liaison sur un site à ou près du site de la picrotoxine du récepteur GABA-A de même que sur un site distinct du récepteur propre aux lactones et aux thiolactones (le site des butyrolactones) (D.F. Covey et al., Mol. Pharmacol., 1997, 52, 114). Il a été démontré que ces composés ne se lient pas au site de liaison des barbiturates et des benzodiazépines du récepteur GABA-A (D.F. Covey et al., Neuropharmacology, 1996, 35, 123).

D'un autre côté, il n'y a pas eu, dans la littérature, de suggestions ou d'indications que les dialkyl-1,4-butyrolactones et thiobutyrolactones interagissent avec le site de reconnaissance du loreclézole anticonvulsivant (**10**) du récepteur GABA-A. L'évidence indirecte suggère que ce n'est pas le cas puisque l'α-EMTBL (**48**) stimule les récepteurs GABA-A composés seulement de sous-unités α (D.F. Covey et al., Neuropharmacology, 1996, 35, 123), alors qu'il est connu que le site de liaison du loreclézole est présent seulement sur les récepteurs contenant les sous-unités β₂ et β₃ (P.B. Wingrove, K.A. Wafford, C. Bain, P.J. Whiting, Proc. Natl. Acad. Sci. USA, 1994, 91, 4569).

Il est ainsi apparent que la présence d'une fonction amine substituée à la position C-3 des 2,2-dialkyl-1,4-butyrolactones, objet de la présente invention, conduit à une augmentation plus importante des effets du GABA que les molécules analogues décrites n'ayant pas cette fonctionnalité. Il apparaît aussi qu'au moins dans certains cas, certains des composés de l'invention peuvent agir par le nouveau site de liaison du loreclézole sur le récepteur GABA-A. Ces deux propriétés donnent aux composés de l'invention une valeur thérapeutique potentiellement grande.

La présente invention concerne donc les composés de formule générale : 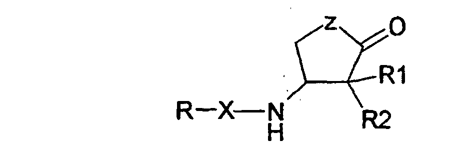 dans laquelle :
Z représente un atome de soufre ou d'oxygène,
les groupes R1 et R2, qui peuvent être identiques ou différents l'un de l'autre, représentent chacun un groupe alkyle ou un groupe alcényle
X représente un CO, un CO₂, un SO ou un SO₂
et le groupe R représente un groupe alkyle, aryle, alcényle ou aralkyle à la condition que lorsque Z représente un atome d'oxygène, X un SO₂ et R un groupe 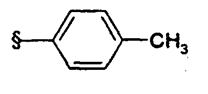
R1 et R2 ne représentent pas tous les deux le groupe méthyl.

Les composés préférés de la présente invention sont tels que Z représente un atome d'oxygène. En effet, ils ont une activité plus intéressante.

Un groupe particulier de composés selon l'invention est constitué par les composés de formule générale : 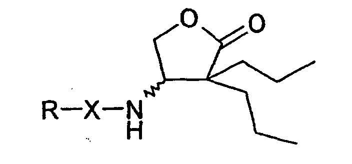 dans laquelle :
X représente un CO, un CO₂, un SO ou un SO₂
et le groupe R représente un groupe alkyle, aryle, alcényle ou aralkyle.

Parmi les composés préférés de l'invention, on peut citer le composé de formule :

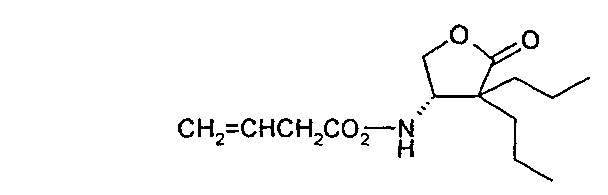

Par le terme de groupe alkyle, on entend les groupes alkyles de 1 à 8 atomes de carbones, linéaires ou ramifiés, substitués ou non substitués. Des exemples préférés de groupes alkyles sont les groupes CH₃, CH₂CH₃, CH₂CH₂CH₃ et C(CH₃)₃.

Par le terme de groupe alcényle, on entend les groupes alcényles de 1 à 6 atomes de carbones, linéaires ou ramifiés, substitués ou non substitués. Un exemple préférés de groupes alcényles est CH₂CH=CH₂.

Par le terme de groupes aryles on entend des cycles aromatiques ayant au moins 3 atomes de carbones, substitués ou non substitués, ayant un seul ou plusieurs noyau aromatiques. Les cycles aromatiques peuvent être accolés ou fusionnés. Des exemples de cycles aromatiques préférés sont les phényles, naphtyles. Des exemples de substituants préférés de ces cycles sont des groupes alkyles tel que CH₃, des atomes d'halogènes tel que Cl, des groupes alkyles halogénés tel que CF₃, des groupes du type OCH₃ et des amines tel que NH₂ ou N(CH₃)₂.

Par le terme de groupes aralkyles on entend des groupes aryles, définis comme ci-dessus, liés au groupe CO ou SO ou CO₂ ou SO₂ par l'intermédiaire d'un groupe alkyle défini comme précédemment. Un exemple préféré de groupe aralkyle est le groupe CH₂Ph.

Les composés selon l'invention possèdent tous un centre d'asymétrie et peuvent donc exister sous forme d'isomères optiques. La présente invention comprend aussi bien ces isomères soit séparément soit en tant que mélange.

La présente invention concerne également le mode de préparation des composés de formule générale 1 suivante: 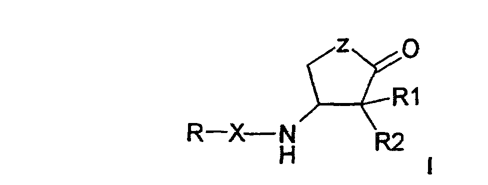 dans laquelle :
Z représente un atome de soufre ou d'oxygène,
les groupes R1 et R2, qui peuvent être identiques ou différents l'un de l'autre, représentent chacun un groupe alkyle ou un groupe alcényle
X représente un CO, un CO₂, un SO ou un SO₂
et le groupe R représente un groupe alkyle, aryle, alcényle ou aralkyle qui peut-être le suivant :
   l'amine du composé de formule générale : 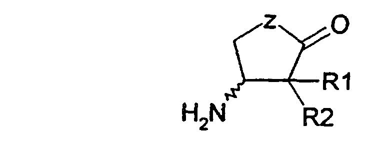
dans laquelle :
Z représente un atome de soufre ou d'oxygène, de préférence un atome d'oxygène,
et les groupes R1 et R2, qui peuvent être identiques ou différents l'un de l'autre, représentent chacun un groupe alkyle ou un groupe alcényle est traitée par un réactif approprié pour obtenir les composés selon la présente invention. De façon préférentielle, ce réactif fait partie de la famille des chlorures d'acyle, chlorures de sulfonyle ou chloroformiates

Ce procédé peut comporter une étape préalable d'hydrogénolyse pour enlever le groupement benzyle du composé de formule générale : 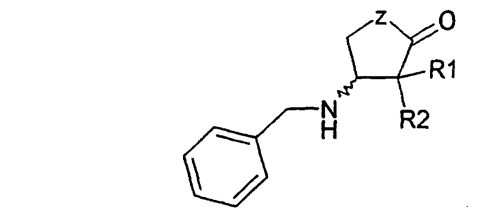 dans laquelle :
Z représente un atome de soufre ou d'oxygène, de préférence un atome d'oxygène,
et les groupes R1 et R2, qui peuvent être identiques ou différents l'un de l'autre, représentent chacun un groupe alkyle ou un groupe alcényle.

Cette étape peut être précédée par une étape d'alkylation en position C-2 du composé de formule générale : 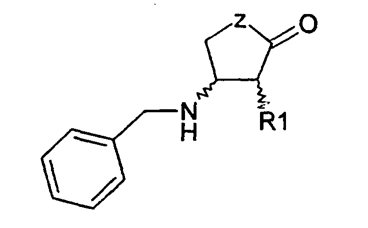 dans laquelle :
Z représente un atome de soufre ou d'oxygène, de préférence un atome d'oxygène,
et le groupe R1 représente un groupe alkyle ou un groupe alcényle
par traitement avec une base forte suivie de l'addition d'un agent alkylant de formule générale R2X dans laquelle R2 représente un groupe alkyle, alcényle ou aralkyle.

Cette étape peut encore être précédée par une étape de monoalkylation en position C-2 de la 3-benzylaminolactone de formule générale : 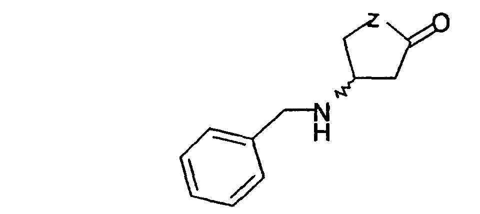 dans laquelle :
Z représente un atome de soufre ou d'oxygène, de préférence un atome d'oxygène,
par traitement avec une base forte suivie de l'addition d'un agent alkylant de formule générale R1X dans laquelle R1 représente un groupe alkyle, alcényle ou aralkyle.

Cette étape peut encore être précédée par une étape d'addition 1, 4 de type Michaël de la benzylamine sur une butyrolactone-1, 4 ou une thiobutyrolactone-1, 4 insaturée pour donner la 3-benzylaminolactone correspondante.

Dans un procédé de préparation préféré selon la présente invention le produit de départ permettant d'obtenir, comme à l'étape précédente, la 3-benzylaminolactone correspondante optiquement pure est un acide aspartique D ou L. Les composés obtenus à la fin de ce procédé, après les 5 étapes décrites précédemment, sont alors optiquement purs.

Dans un autre exemple de procédé de préparation, la base forte utilisée pour réaliser les alkylations est l'hexaméthyldisilazide de lithium.

La présente invention concerne également les compositions pharmaceutiques comprenant à titre de principe actif un composé de formule générale suivante : 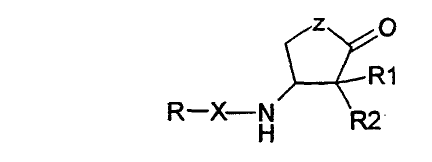 dans laquelle :
Z représente un atome de soufre ou d'oxygène,
les groupes R1 et R2, qui peuvent être identiques ou différents l'un de l'autre, représentent chacun un groupe alkyle ou un groupe alcényle
X représente un CO, un CO₂, un SO ou un SO₂
et le groupe R représente un groupe alkyle, aryle, alcényle ou aralkyle
et un excipient approprié. Ces compositions peuvent être formulées pour l'administration aux mammifères, y compris l'homme. La posologie varie selon le traitement et selon l'affection en cause. Ces compositions sont réalisées de façon à pouvoir être administrées par la voie digestive ou parentérale.

Dans les compositions pharmaceutiques de la présente invention pour l'administration orale, sublinguale, sous-cutanée, intramusculaire, intraveineuse, transdermique, locale ou rectale, l'ingrédient actif peut être administré sous formes unitaires d'administration, en mélange avec des supports pharmaceutiques classiques, aux animaux ou aux êtres humains. Les formes unitaires d'administration appropriés comprennent les formes par voie orale telles que les comprimés, les gélules, les poudres, les granules et les solutions ou suspensions orales, les formes d'administration sublinguale et buccale, les formes d'administration sous-cutanée, intramusculaire, intraveineuse, intranasale ou intraoculaire et les formes d'administration rectale.

Lorsque l'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir peut contenir l'ingrédient actif conjointement avec un édulcorant, un antiseptique, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, de même qu'avec des correcteurs du goût ou des édulcorants.

Pour une administration rectale, on recourt à des suppositoires qui sont préparés avec des liants fondant à la température rectale, par exemple du beurre de cacao ou des polyéthylèneglycols.

Pour une administration parentérale, intranasale ou intraoculaire, on utilise des suspensions aqueuses, des solutions salines isotoniques ou des solutions stériles et injectables qui contiennent des agents de dispersion et/ou des agents mouillants pharmacologiquement compatibles.

Le principe actif peut être formulé également sous forme de microcapsules, éventuellement avec un ou plusieurs supports additifs.

La présente invention concerne également l'utilisation des composés de formule générale suivante : 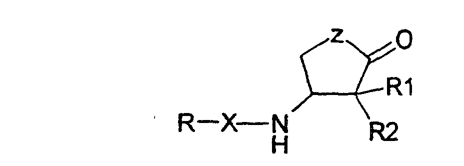 dans laquelle :
Z représente un atome de soufre ou d'oxygène,
les groupes R1 et R2, qui peuvent être identiques ou différents l'un de l'autre, représentent chacun un groupe alkyle ou un groupe alcényle
X représente un CO, un CO₂, un SO ou un SO₂
et le groupe R représente un groupe alkyle, aryle, alcényle ou aralkyle
comme stimulant de l'activité de l'acide γ-aminobutyrique agissant via le récepteur GABA-A du système nerveux central.

Les composés selon l'invention de formule générale suivante : 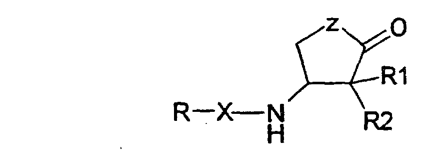 dans laquelle :
Z représente un atome de soufre ou d'oxygène,
les groupes R1 et R2, qui peuvent être identiques ou différents l'un de l'autre, représentent chacun un groupe alkyle ou un groupe alcényle
X représente un CO, un CO₂, un SO ou un SO₂
et le groupe R représente un groupe alkyle, aryle, alcényle ou aralkyle
et les compositions pharmaceutiques les comprenant peuvent être utilisés comme médicament, en particulier pour le traitement des troubles nerveux. Ces troubles sont de préférence du type épilepsie, anxiété, dépression, troubles du sommeil, attaques de panique, contractions musculaires, douleur, dépendance à l'alcool ou aux benzodiazépines ou comportements psychotiques.

### SYNTHESE

### Préparation des composés racémiques

En utilisant la méthode décrite par Perlmutter (M.P. Collis, P. Perlmutter, Tetrahedron: Asymmetry, 1996, 7, 2117), l'addition 1-4 de type Michaël de la benzylamine sur la 2-(5H)-furanone commerciale **12** dans du méthanol donne un mélange racémique de la 3-benzylamino-1,4-butyrolactone **13** (schéma 1). Ce dernier est traité tout d'abord avec de l'hexaméthyldisilazide de lithium dans du THF à -78°C et ensuite avec un halide d'alkyle en présence de HMPA pour donner le mélange *cis*/*trans* de la 2-monoalkyl-3-benzylamino-1,4-butyrolactone **14** qui peut être séparé par colonne chromatographique sur gel de silice. On utilise comme agents alkylants typiques l'iodure de méthyle, le chlorure de p-méthoxybenzyle, le 2-bromoéthylbenzène.

Dans le cas du bromure d'allyle, le produit principal obtenu est le dérivé 2,2-diallyle **16**. Les dérivés monoalkyles **14** peuvent plus tard être alkylés en dérivés dissymétriques 2,2-dialkylés **15**.

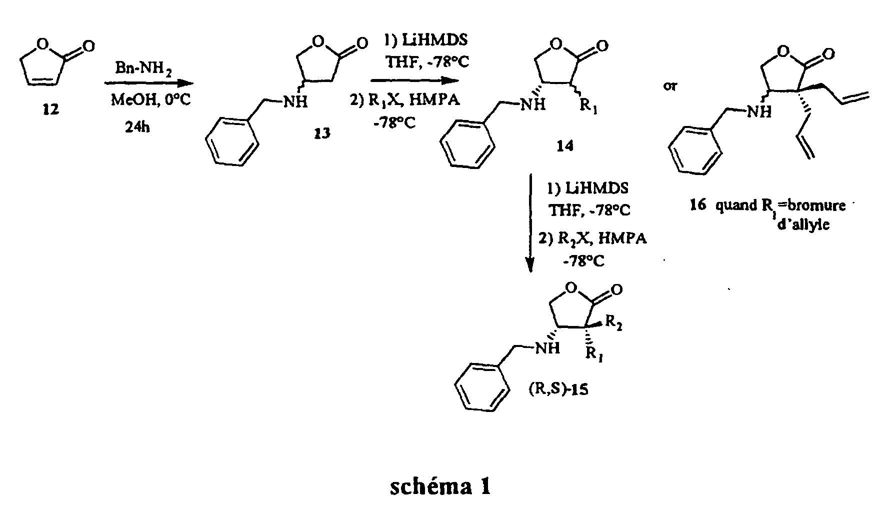

Les dérivés monalkylés **14** ou dialkylés **15** sont soumis à une hydrogénolyse catalytique dans de l'éthanol à 40 p.s.i. en présence de 10 % de palladium sur du carbone pour donner les dérivés instables 3-amino correspondants **17** ou **18,** respectivement (schéma 2). Ces derniers sont immédiatement traités dans du dichlorométhane en présence de triéthylamine et de DMAP avec des chlorures d'alkyle, d'alcényle ou d'arylsulfonyle, des chlorures d'alkyloxycarbonyle, d'alcénoxycarbonyle or d'aryloxycarbonyle ou des chlorures d'acide d'alkyle d'alcényle ou d'aryle pour donner les dérivés N-substitués correspondants **19** ou **20** respectivement. Alternativement, les composés **14** ou **15** peuvent être traités avec les mêmes réactifs pour donner les dérivés N,N-disubstitués **21** ou **22,** respectivement.

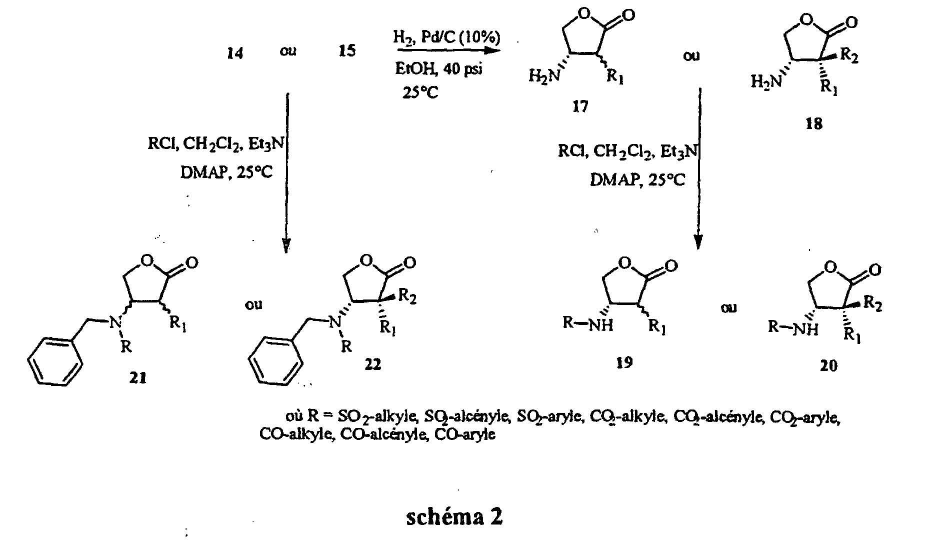

Dans le cas spécial du dérivé diallyle **16,** l'hydrogénolyse du groupe N-benzyle conduit aussi à la réduction des doubles liaisons pour donner la 2,2-dipropyl-3-amino-1,4-butyrolactone **18** (R1=R2=propyle) qui est sulfonylée ou N-acylée comme ci-dessus pour donner les composés **20** (R1 = R2 = propyle).

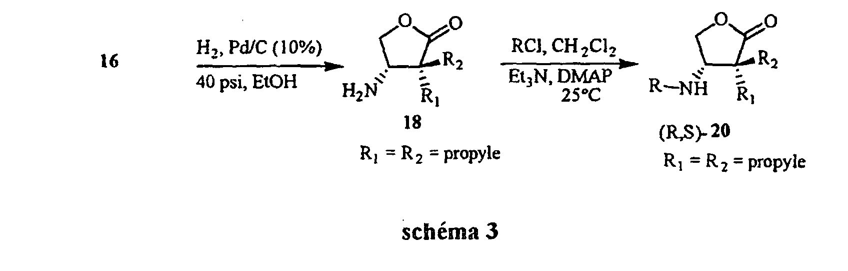

### Isolement des énantiomères purs des composés selon l'invention.

### a) par HPLC sur une colonne chirale,

Les mélanges racémiques des composés *R,S*-**20** peuvent être séparés par HPLC sur colonne chirale. Par exemple, l'application de 40 mg du racémique **30** sur une colonne OD chirale et l'élution avec 9:1 d'hexane/2-propanol résulte dans l'isolement de 12 mg d'isomère *R* (temps de rétention = 12,3 min; [α]_{D} = -0,28 (CHCl₃)) et de 13 mg d'isomère *S* (temps de rétention = 13,6 min; [α]_{D} = +0,24 (CHCl₃)), les deux fractions ayant une pureté énantiomérique de plus de 95 %.

### b) par synthèse énantiospécifique à partir de l'acide aspartique

La 3-benzylamino-1,4-butyrolactone **13** de départ peut être préparée dans une forme énantiomériquement pure en partant de l'acide aspartique L ou D et en employant une méthodologie décrite précédemment (G.J. McGarvey, et al., J. Amer. Chem. Soc., 1986, 108, 4943). Ainsi, le traitement de l'acide aspartique D D-**23** dans un mélange d'éther et de 3N d'hydroxyde de sodium avec du chlorure de benzyloxycarbonyle donne le dérivé N-benzyloxycarbonyle **24** (Schéma 4). Le traitement de ce dernier avec de l'anhydride acétique produit l'anhydride **25** qui est sélectivement réduit en (*R*)-3-(benzyloxycarbonylamino)-1,4-butyrolactone ((*R*)-**26**). L'enlèvement du groupe bloquant Cbz par hydrogénolyse et l'alkylation réductrice de l'amine résultante avec du benzaldéhyde donne la benzylamine (*R*)-**13**. Le traitement de cette dernière comme précédemment avec de l'hexaméthyldisilazide de lithium et un excès de bromure d'allyle (schéma 1) donne alors le composé (*R*)-**16** qui peut être transformé en composé (*R*)-**20** en utilisant la même série de réactions utilisée pour préparer le composé racémique **20** (schéma 3).

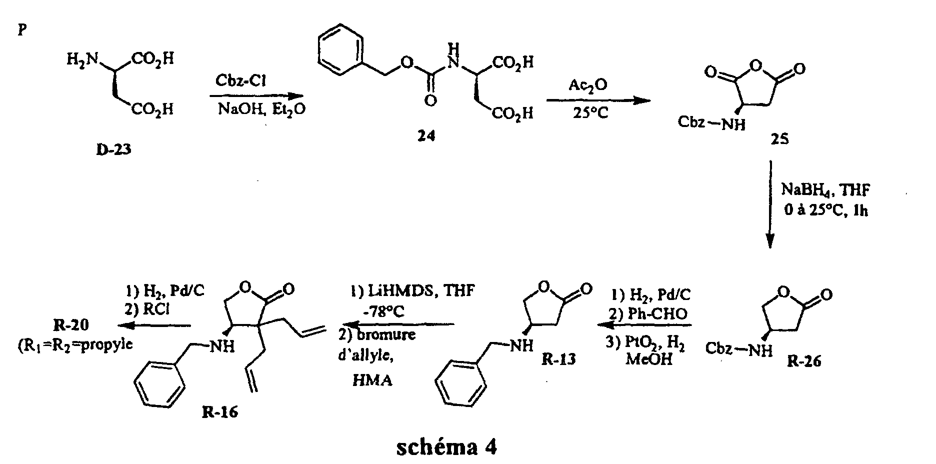

L'application de ce schéma réactionnel à l'acide aspartique L (L-**23**) donne alors accès à l'énantiomère (*S*)-**20** (schéma 5).

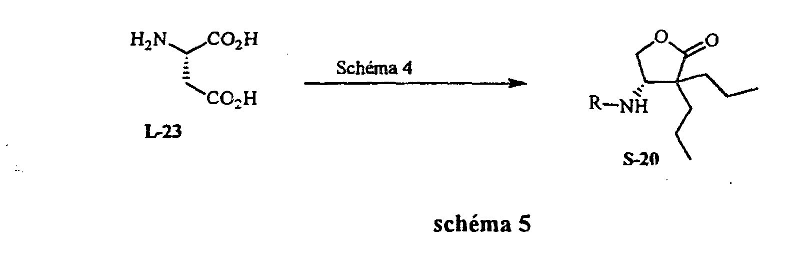

### ETUDES DE LIAISON SUR LE RECEPTEUR

Les composés synthétisés sont testés *in vitro* pour leur capacité à se lier à différents sites sur le récepteur GABA-A, incluant le site du GABA lui-même (par des études de déplacement du H³-muscimol), le site des benzodiazépines (par déplacement du H³-flunitrazépam), le site de la picrotoxine (par déplacement du S³⁵-TBPS). Brièvement, des membranes gelées provenant du cervelet ou du cerveau sans cervelet sont dégelées, centrifugées et remises en suspension dans 50 mM de tampon Tris-citrate, pH 7,4, à une concentration en protéines d'environ 1 mg/ml. Les membranes (0,5 ml) sont alors incubées dans un total de 1 ml de solution contenant 50 mM de tampon Tris-citrate, pH 7,4, 150 mM de NaCl et 2 nM de [H³]flunitrazépam ou 2 nM de [H³]muscimol en absence ou en présence de concentrations variées du composé à étudier ou de 10 µM de diazépam ou de 10 µM de GABA, pendant 90 min à 4°C, respectivement. Pour la liaison [S³⁵]TBPS, les membranes sont incubées dans un total de 1 ml de solution contenant 50 mM de tampon Tris-citrate, pH 7,4, 200 mM de NaBr et 2 nM de [S³⁵]TBPS en absence ou en présence de concentrations variées du composé à étudier ou de 10 µM de TBPS ou de picrotoxinine pendant 180 min à température ambiante (W. Sieghart, A. Schuster, Biochem. Pharmacol., 1984, 33, 4033 et J. Zezula et al., Eur. J. Pharmacol., 1996, 301, 207).

Les membranes sont alors filtrées à travers des filtres Whatman GF/B. Quand la liaison du [H³]flunitrazépam ou du [H³]muscimol est étudiée, les filtres sont rincés deux fois avec 5 ml d'une solution tampon de 50 mM de Tris-citrate glacé. Lorsque la liaison du [S³⁵]TBPS est étudiée, les filtres sont rincés trois fois avec 3,5 ml de cette solution tampon. Les filtres sont transférés dans des fioles à scintillation et soumises au comptage à scintillation après addition de 3,5 ml de fluide de scintillation. La liaison non-spécifique déterminée en présence de 10 µM de diazépam, de 10 µM de GABA ou de 10 µM de TBPS est soustraite du total de la liaison du [H³]flunitrazépam, du [H³]muscimol, ou du [S³⁵]TBPS respectivement pour obtenir la liaison spécifique.

### ETUDES ELECTROPHYSIOLOGIQUES

Les composés synthétisés sont aussi étudiés pour leur habilité à provoquer l'ouverture du canal du récepteur GABA-A ou à moduler allostériquement les courants provoqués par le GABA. Dans ce but, les récepteurs recombinants GABA-A sont exprimés dans des ovocytes de Xénope. Brièvement, les ovocytes de Xenopus laevis sont préparés, injectés, défolliculés et les courants sont enregistrés de la façon décrite (E. Sigel, J. Physiol., 1987, 386, 73 et E. Sigel, R. Baur, G. Trube, H. Möhler, P. Malherbe, Neuron, 1990, 5, 703). Les ovocytes sont injectés avec 50 nl d'ARNc dissous dans 5 mM de K-Hepes (pH 6,8). Cette solution contient les transcripts codants pour les différentes sous-unités à une concentration de 10 nM pour α₁, 10 nM pour β₂ et 50 nM pour γ₂. Les transcripts d'ARN sont synthétisés à partir de plasmides linéarisés encodant la protéine désirée en utilisant la trousse de message machine (Ambion) selon la recommandation des fabricants. Une queue poly(A) d'environ 300 résidus est ajoutée aux transcripts en utilisant le poly(A) polymérase de levure (USB ou Amersham). Les combinaisons d'ARNc sont co-precipitées dans de l'éthanol et stockées à -20°C. Les transcripts sont quantifiés sur des gels agarose après avoir été colorés avec le colorant ARN Rouge Radiant (Bio-Rad) en comparant les intensités des colorations avec différentes quantités de marqueurs de poids moléculaire (ARN-Ladder, Gibco-BRL). Les expériences électrophysiologiques sont réalisées par la méthode de pince de tension à deux électrodes à un potentiel de fixation de -80 mV. Le milieu contient 90 mM de NaCl, 1 mM de KCl, 1 mM de MgCl₂, 1 mM de CaCl₂ et 10 mM de Na-Hepes (pH 7,4). Du GABA est appliqué pendant 20 s et une période de lavage de 4 min est permise pour assurer le complet rétablissement de la désensibilisation. Le système de perfusion est nettoyé entre les applications des composés par lavage avec du sulfoxide de diméthyle pour éviter la contamination. Les composés sont appliquées à une concentration de 100 microM en absence de GABA pour voir s'ils peuvent agir comme agoniste du canal. Pour étudier la modulation allostérique, du GABA est tout d'abord appliqué seul puis en combinaison avec soit 0,1 microM soit 100 microM de composés.

### RESULTATS PHARMACOLOGIQUES

Tous les composés synthétisés sont testés *in vitro* pour leur habilité à déplacer le flunitrazépam tritiaté (H³-Flu, ligand sélectif du site de liaison des benzodiazépines du récepteur GABA-A), S³⁵-TBPS (sélectif pour le site de liaison de la picrotoxine) et le muscimol tritiaté (sélectif pour le site de liaison du GABA). Ces composés sont aussi testés pour leur habilité à empêcher ou à stimuler les courants provoqués par le GABA dans des ovocytes de grenouilles exprimant le sous-type α₁β₂γ₂ du récepteur du GABA. Les résultats détaillés pour tous les composés sont montrés dans les tableaux 1a, 1b, 1c et 1d.

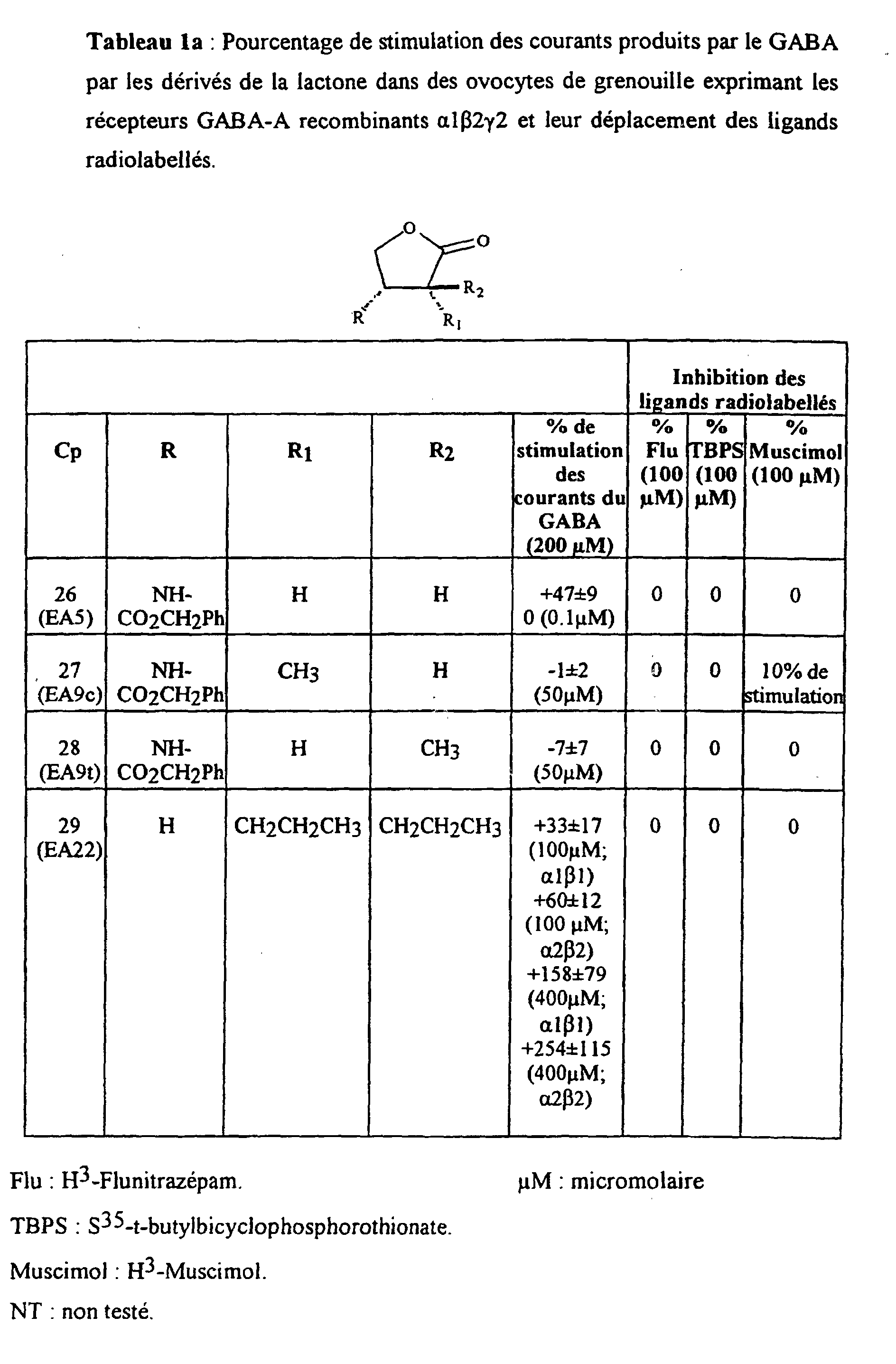

**Tableau 1b**

| | | | | | **Inhibition des ligands radiolabellés** | | |
|---|---|---|---|---|---|---|---|
| **Cp** | **R** | **R**_{**1**} | **R**_{**2**} | **% de stimulation des courants du GABA (200 µM)** | **% Flu** **(100 µM)** | **% TBPS (100 µM)** | **% Muscimol** **(100 µM)** |
| 30 (EA18) | NH-CO₂₋ CH₂Ph | CH₂CH₂CH₃ | CH₂CH₂CH₃ | +51±30 +250 (50µM) +140±57 (10 µM) +15±3 (1µM) | 0 | 55% 0 (10µM) | 0 |
| 31 (EA31) | NH-SO₂-Ph-p-Cl | CH₂CH₂CH₃ | CH₂CH₂CH₃ | +23±8 (5µM) (insoluble à >5 µM) | 25% stimulation | IC₅₀= 10µM | 0 |
| 32 (EA32) | NH-SO₂-Ph-p-CF₃ | CH₂CH₂CH₃ | CH₂CH₂CH₃ | N.T. | 15-20% stimulation | 100% (10µM, partie antérieure du cerveau) inhibition incomplète dans le cervelet | 0 |
| 33 (EA33) | NH-SO₂-Ph-p-OCH₃ | CH₂CH₂CH₃ | CH₂CH₂CH₃ | N.T. | faible stimulation | IC₅₀= 50 µM (coopérativité) | 0 |
| 34 (EA34) | NH-CO-Ph | CH₂CH₂CH₃ | CH₂CH₂CH₃ | +420±33 +207±17 (20 µM) +17±3 (2µM) | faible stimulation | IC₅₀= 100 µM (coopérativité) | 0 |

**Tableau 1c**

| | | | | | **Inhibition des ligands radiolabellés** | | |
|---|---|---|---|---|---|---|---|
| **Cp** | **R** | **R**_{**1**} | **R**_{**2**} | **% de stimulation des courants du GABA (200 µM)** | **% Flu (100 µM)** | **% TBPS (100 µM)** | **% Muscimol** **(100 µM)** |
| 35 (EA35) | NH-CO-OCH₂-H=CH₂ | CH₂CH₂CH₃ | CH₂CH₂CH₃ | +765±61 +229±14 (20 µM) +26±5 (2µM) | faible stimulation | IC₅₀= 100 µM (coopérativité) | 0 |
| 36 (EA36) | NH-CO-OCH₂CH₃ | CH₂CH₂CH₃ | CH₂CH₂CH₃ | N.T. | 0 | IC₅₀ > 100 µM | 0 |
| 37 (EA44) | NH-CO₂-C(CH₃)₃ | CH₂CH₂CH₃ | CH₂CH₂CH₃ | N.T. | | | |
| 16 (EA17) | NH-CH₂Ph | CH₂CH=CH₂ | CH₂CH=CH₂ | +115±30 | 10% stimulation | 62% | |
| 38 EA20c) | NH-CH₂Ph | CH₂CH₂Ph | H | N.T. | | | |
| 39 EA20t) | NH-CH₂Ph | H | CH₂CH₂Ph | N.T. | | | |
| 40 EA19a) | NH-CH₂Ph | CH₃ | CH₂Ph-p-OCH₃ | N.T. | | | |
| 41 EA19b) | NH-CH₂Ph | CH₂Ph-p-OCH₃ | CH₃ | N.T. | | | |
| 42 EA12t) | N(CH₂ Ph)-CO-OCH₂Ph | H | CH₃ | +3±10 | 0 | 52% 15% (10µM) | 0 |

**Tableau 1d**

| | | | | | **Inhibition des ligands radiolabellés** | | |
|---|---|---|---|---|---|---|---|
| **Cp** | **R** | **R**_{**1**} | **R**_{**2**} | **% de stimulation des courants du GABA (200 µM)** | **% Flu (100 µM)** | **% TBPS (100 µM)** | **% Muscimol (100 µM)** |
| 43 EA12c) | N(CH₂Ph) -CO-OCH₂Ph | CH₃ | H | -3±3 | 30% 20% (10 µM) | 60% 35% (10 µM) | 0 |
| 44 (EA56) | NH-SO₂-Ph-p-NH₂ | CH₂CH₂CH₃ | CH₂CH₂CH₃ | | | | |
| 45 (EA54) | NH-SO₂-Ph-p-NHY (Y= CO₂CH₂ CH=CH₂) | CH₂CH₂CH₃ | CH₂CH₂CH₃ | | | | |
| 46 (EA58) | NHSO₂-N(CH₃)₂ | CH₂CH₂CH₃ | CH₂CH₂CH₃ | | | | |
| 47 (EA57) | NH-SO₂-(2-thiophène) | CH₂CH₂CH₃ | CH₂CH₂CH₃ | | | | |
| 48 (EA46) | α-Ethyl, α-methyl-γ-thiobutyrolactone (EMTBL) | | | | | | |

Les composés les plus actifs sont résumés dans les tableaux 2a et 2b.

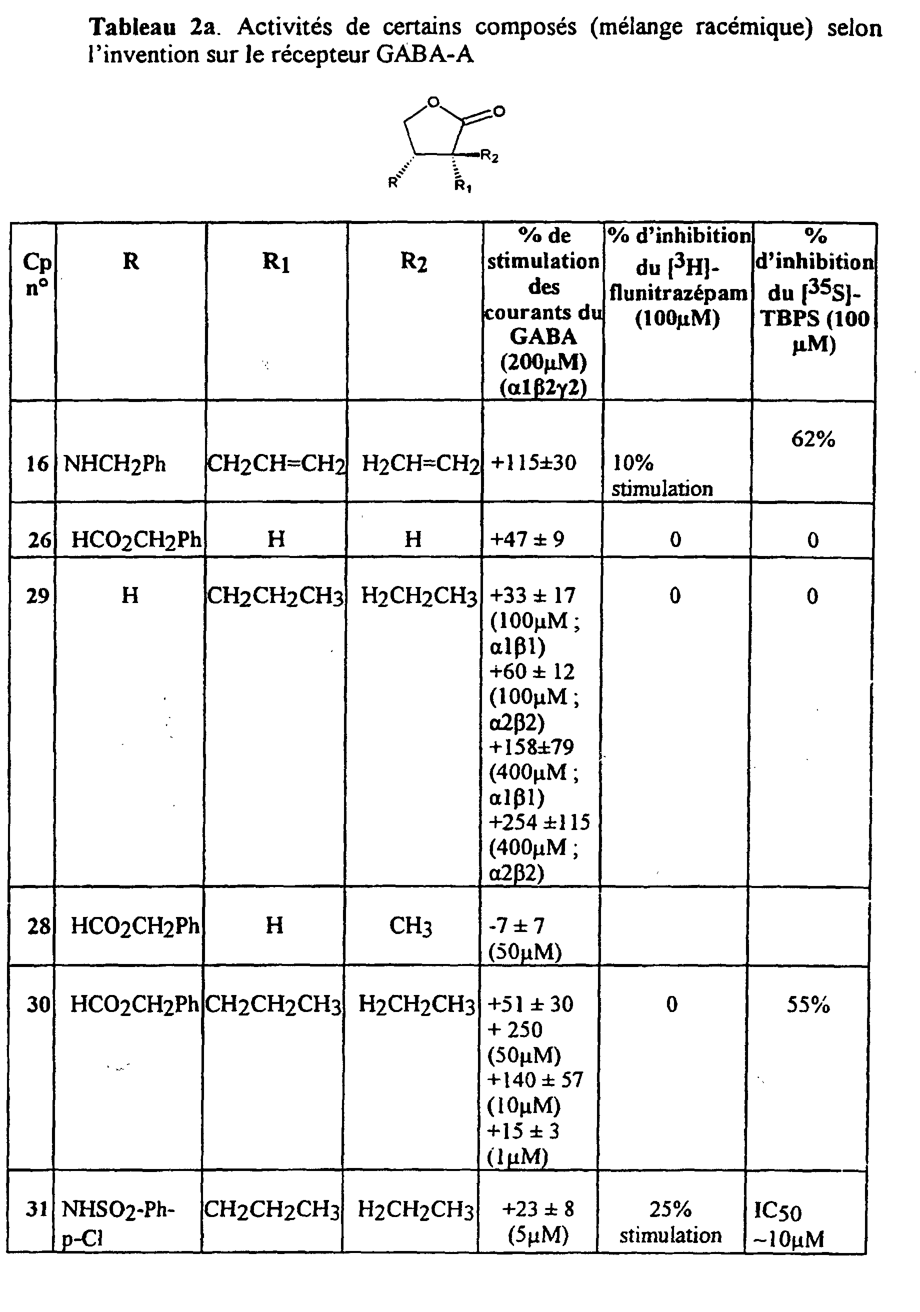

**Tableau 2b**

| **Cp n°** | **R** | **R**_{**1**} | **R**_{**2**} | **% de stimulation des courants du GABA (200µM)** **(α1β2γ2)** | **% d'inhibition du [**^{**3**}**H]- flunitrazépam (100µM)** | **% d'inhibition du [**^{**35**}**S]- TBPS (100 µM)** |
|---|---|---|---|---|---|---|
| 34 | NHCOPh | CH₂CH₂CH₃ | H₂CH₂CH₃ | + 420 ± 30 +207 ± 17 (20µM) +17± 3 (2µM) | faible stimulation | IC₅₀ ∼100µM |
| 35 | H-CO₂-H₂CH=CH₂ | CH₂CH₂CH₃ | H₂CH₂CH₃ | +765 ± 61 +229 ± 14 (20µM) +26± 5 (2µM) | faible stimulation | IC₅₀ ∼100µM |
| **Diazépam** | | | | +150 à +300 (1µM) | | |
| **α-EMTBL** | | | | +152±6 (1mM) +20-50 (200 µM) 0(100µM) | 0 | IC₅₀ =500µM |

Ces exemples de composés et les résultats obtenus avec eux sont indiqués à titre non limitatif et illustrent l'invention.

Les conclusions qui peuvent être tirées de l'étude activité-fonction sont les suivantes :
- Les composés les plus actifs (c'est-à-dire, ceux produisant la plus grande stimulation des courants produits par le GABA) sont ceux ayant simultanément une substitution gem-dialkyle à la position α (ou 2) et une amide secondaire, une sulfonamide ou un carbamate à la position β (ou 3). Ceux-ci sont représentés respectivement par la phénylcarboxamide **34** (420% de stimulation des courants produits par le GABA à 200 µM; 207% de stimulation à 20 µM; 17% de stimulation à 2 µM), la p-chlorophénylsulfonamide **31** (23% de stimulation à 5 µM (insoluble à de plus hautes concentrations)) et le carbamate d'allyle **35** (765% de stimulation à 200 µM, 229% à 20 µM et 26% à 2 µM). Ce dernier est le composé synthétisé le plus actif En comparaison, l'α-éthyl-α-méthylthiobutyrolactone (α-EMTBL, **48**), la butyrolactone anticonvulsivante la plus active décrite jusqu'à présent, ne produit pas de stimulation des courants du GABA à 100 µM. Une concentration de 200 µM d'α-EMTBL est requise pour produire une stimulation de 20 % (G.C. Mathews et al., Neuropharmacology, 1996, 35, 123). Les composés de la présente invention (par exemple **35**) sont donc jusqu'à 100 fois plus puissants que l'α-EMTBL pour stimuler les courants produits par le GABA.
- L'importance de la présence simultanée du substituant amine à la position β et des substituants dialkyles à la position α est démontrée par les faibles activités stimulantes du courant du GABA des composés **26** (seulement un substituant amine sur le noyau de la lactone), **29** (seulement des substituants dialkyles) et **28** (seulement un substituant amine et un substituant alkyle).
- Aucun des composés de la présente invention ne déplace le flunitrazépam tritiaté de son site de liaison, indiquant ainsi que ces composés ne se lient pas au site de liaison des benzodiazépines du récepteur GABA-A. Dans certains cas (par exemple, **31, 34, 35**) une faible stimulation de la liaison du flunitrazépam est observée, probablement due aux interactions allostériques provenant de la liaison de ces composés à d'autres sites sur le récepteur.
- Une faible interaction de certains de ces composés (par exemple **30**, **31**, **34**, **35**) avec le site de liaison de la picrotoxine du récepteur GABA-A est démontrée par le déplacement du S³⁵-TBPS avec des IC₅₀ de l'ordre de 10 à 100 µM. Il n'y a pas de corrélation apparente entre les affinités avec le site de liaison du TBPS et la capacité à stimuler les courants produits par le GABA.
- Aucun des composés de la présente invention ne déplace le H³-muscimol (tableau 1), indiquant ainsi que ces composés ne se lient pas au site de reconnaissance du GABA sur le récepteur GABA-A.
- Comme il est montré dans le tableau 3, l'activité est différente suivant la stéréochimie du substituant amine du noyau de la lactone. Ainsi le composé R-30 (obtenu soit par séparation du mélange racémique sur une colonne chirale soit par synthèse énantiospécifique à partir de l'acide aspartique D, schéma 4) est deux fois plus puissant que l'isomère *S* correspondant.

**Tableau 3 :**

| Stimulation des courants produits par le GABA par interaction des énantiomères du composé **30** avec les récepteurs recombinants GABA-A α1β2γ2 | | |
|---|---|---|
| **isomère** | **100 µM** | **10 µM** |
| **R-30** | +279±94% | + 51 ±94% |
| **S-30** | + 104 ± 7 % | + 17 ± 5 % |

Alors que les composés de l'invention apparemment n'interagissent pas avec les sites de reconnaissance des benzodiazépines ou du GABA du récepteur GABA-A et se lient seulement faiblement avec le site de reconnaissance de la picrotoxine/TBPS, les expériences avec les récepteurs GABA-A recombinants ayant différentes compositions de sous-unités suggèrent qu'au moins certains des composés peuvent interagir avec le site de liaison du loreclézole. Ainsi, on observe que le composé *R*-**30** (100 µM) produit une stimulation 5 fois plus grande des courants du GABA dans les récepteurs contenant les sous-unités β₂ par rapport aux récepteurs ne portant que la sous-unité β₁ (tableau 4). A 10 µM, le composé *R*-**30** ne produit qu'une stimulation dans les récepteurs contenant la sous-unité β₂. Le loreclézole anticonvulsivant similairement augmente l'activité du récepteur GABA-A en interagissant avec un site présent sur les récepteurs contenant la sous-unité β₂ (de même que β₃) mais non présent sur les récepteurs contenant la sous-unité β₁ (P.B. Wingrove, K.A. Wafford, C. Bain, P.J. Whiting, Proc. Natl. Acad. Sci. USA, 1994, 91, 4569). De plus l'anticonvulsivant α-EMTBL est capable de stimuler les récepteurs contenant seulement les sous-unités α, suggérant que l'α-EMTBL n'agit pas sur le site de liaison du loreclézole. Les composés de la présente invention semblent donc stimuler l'activité du GABA en agissant sur un site différent de celui de l'α-EMTBL et des composés apparentés en dépit de la ressemblance structurelle apparente de ces deux classes de composés.

**Tableau 4 :**

| Stimulation des courants produits par le GABA par le composé **R-30** sur les récepteurs recombinants GABA-A ayant une sous-unités β1 ou β2 | | |
|---|---|---|
| **Sous-unités** | **10 µM** | **100 µM** |
| α1β1 | - 5 ± 11 % | + 92±28% |
| α2β2 | + 78 ± 4 % | + 588 ± 166 % |

Les exemples suivants, donnés à titre non limitatif illustrent l'invention.

### PROCEDE DE SYNTHESE

### La (R,S)-3-benzylamino-1,4,-butyrolactone 13

Une solution de 2-(5H)furanone (2,5 g, 2,1 ml, 29,7 mmol) dans du méthanol (3 ml) est refroidie à 0°C et traitée avec de la benzylamine (3,82 g, 3,9 ml, 35,7 mmol). La solution résultante est agitée à 0°C pendant 24 heures. Le solvant est évaporé et le résidu est purifié par chromatographie flash (éluant 1/1 : EtOAc/hexanes). Les fractions contenant la substance de Rf=0,07 sont réunies et concentrées pour donner le composé **13** sous forme d'une huile jaune (3,5 g, 60 %). Analyse élémentaire pour C₁₁H₁₃NO₂ : Calculée, %: C,69,09 ; H, 6,85 ; N, 7,32. Trouvée, % : C,68,94 ; H, 6,82 ; N, 7,22.

### Procédé général d'alkylation de la 3-benzylamino-1,4-butyrolactone [(R,S)-13]

Une solution de lactone **13** dans du THF est ajoutée goutte à goutte à une solution d'hexaméthyldisilazide de lithium (2,2 equiv) dans du THF à -78°C sous atmosphère d'argon sous agitation. Après 30 minutes à cette température, l'électrophile dans du HMPA est alors ajouté par canule dans la solution d'énolate. Le mélange est alors agité pendant le temps spécifié et la réaction est neutralisée par addition d'une solution aqueuse saturée de chlorure d'ammonium. Le mélange est alors extrait plusieurs fois avec CH₂Cl₂, séché (MgSO₄), filtré et concentré sous pression réduite. Les produits bruts sont alors purifiés par chromatographie ainsi qu'il sera décrit individuellement pour chacun d'eux.

### La trans-3-Benzylamino-2-C-méthyl-1,4-butyrolactone (trans-14)

Une solution d'énolate de lithium préparée à partir de la lactone (*R,S*)-**13** (0,366g, 1,916 mmol) à -78°C est traitée avec une solution d'iodométhane (5 équiv, 0,6 ml, 9,58 mmol) dans du HMPA (0,5 ml). Le mélange résultant et agité à -78°C pendant 2 heures. La réaction est alors neutralisée à 0°C pour obtenir une huile jaune. Le produit brut est purifié par chromatographie flash (1/1: EtOAc/hexanes). Les fractions contenant la substance de Rf=0,05 sont réunies et concentrées pour obtenir 0,07g (38%) de *trans-* **14** (R₁= CH₃).
Analyse élémentaire pour C₁₂H₁₅NO₂ : Calculée, % : C, 70,22; H, 7,37; N, 6,82. Trouvée, % : C, 70,04; H, 7,32; N, 6,79.

Les fractions contenant la substance de Rf=0,06 sont réunies et concentrées de façon à obtenir 0,016g (9%) de *cis*-**14** (R₁= CH₃).

### Les cis et trans-3-Benzylamino-2-C-allyl-1,4-butyrolactones cis-14 et trans-14, la 3-Benzylamino-3,3-di-C-allyl-1,4-butyrolactone 16

Une solution d'énolate de lithium préparée à partir de la lactone (*R,S*)-**13** (0,46g, 2,44 mmol) à -78°C est traitée avec une solution de bromure d'allyle (5 équiv, 1,16 ml, 12,2 mmol) dans du HMPA (5 ml). Le mélange résultant est agité à -78°C pendant 2 heures. La réaction est alors neutralisée à 0°C pour obtenir une huile jaune. Le produit brut est purifié par chromatographie flash (1/1: EtOAc/hexanes). Les fractions contenant la substance de Rf=0,4 sont réunies et concentrées pour obtenir 0, 1g (18%) de *trans-* **14** (R₁= allyle).
HRMS calculé pour C₁₄H₁₈NO₂ donne 232,1337. On a trouvé 232,1337. Infrarouge (film): 1772,5 (CO) cm⁻¹. Analyse élémentaire pour C₁₄H₁₇NO₂: Calculée, % : C, 72,70; H, 7,41; N, 6,06. Trouvée, % : C, 72,46; H,7,61; N,6,06.

Les fractions contenant la substance de Rf=0,5 sont réunies et concentrées pour obtenir 0,015g (9%) de *cis-***14** (R₁= allyle).
Infrarouge (film): 1772,3 (CO) cm⁻¹. SM (CI, Isobut) m/z 232 (M+1). HRMS calculé pour C₁₄H₁₈NO₂: 232,1337. On a trouvé 232,1339.

Les fractions contenant la substance de Rf=0,7 sont réunies et concentrées pour obtenir le composé diallyle **16** (0,34g, 52%).
SM (Cl, Isobut.) : m/z 272 (M+1). Infrarouge (film) : 1639,3 (allyle), 1770 (CO lactone), 3338 (NH) cm⁻¹. Analyse élémentaire pour C₁₇H₂₁NO₂ : Calculée, %: C, 75,25; H, 7,80; N, 5,16. Trouvée, % : C, 74,87; H, 7,91; N, 5,08.

### Les cis et trans-3-Benzylamino-2-C-(p-méthoxybenzyl)-1,4-butyrolactones cis-14 et trans-14

Une solution d'énolate de lithium préparée à partir de la lactone (*R,S*)-**13** (0,46g, 2,44 mmol) à -78°C est traitée avec une solution de bromure de p-méthoxybenzyle (5 équiv, 1,65 ml, 12,2 mmol) dans du HMPA (5 ml). Le mélange résultant est agité à -78°C pendant 4 heures. La réaction est alors neutralisée à 0°C pour obtenir une huile jaune. Le produit brut est purifié par chromatographie flash (1/3: EtOAc/hexanes). Les fractions contenant la substance de Rf=0,2 sont réunies et concentrées pour obtenir 0,076g (10%) de *trans*-**14** (R₂= p-méthoxybenzyle). Infrarouge (film): 1512,7, 1611,7, 1771,8 (CO lactone), 3400 (NH) cm⁻¹. SM (CI, isobut.) : m/z 312 (M+1). HRMS calculé pour C₁₉H₂₂NO₃: 312,1599. On a trouvé 312,1595.

Les fractions contenant la substance de Rf=0,3 sont réunies et concentrées pour obtenir 0,05g (7%) de *cis-***14** (R₁= p-méthoxybenzyle).
Infrarouge (film) :1612,3, 1772,3 (CO lactone), 3398 (NH) cm⁻¹.
SM (CI, isobut.) : m/z 312 (M+1). HRMS calculé pour C₁₉H₂₂NO₃: 312,1599. On a trouvé 312,1585.

### Les cis et trans-3-Benzylamino-2-C-(benzyléthyl)-1,4-butyrolactones 38 et 39

Une solution d'énolate de lithium préparée à partir de la lactone (*R,S*)-**13** (0,11g, 0,6 mmol) à -78°C est traitée avec une solution de 2-bromoéthylbenzène (5 equiv, 0,42 ml, 3 mmol) dans du HMPA (1 ml). Le mélange résultant est agité à -78°C pendant 6 heures. La réaction est alors neutralisée à 0°C pour obtenir une huile jaune. Le produit brut est purifié par chromatographie flash (1/1: EtOAc/hexanes). Les fractions contenant la substance de Rf=0,4 sont réunies et concentrées pour obtenir le composé **39** (0,015 g, 8%).
SM (CI, Isobut.) : m/z 296 (M+1). HRMS calculé pour C₁₉H₂₂NO₂: 296,1650. On a trouvé 296,1650.

Les fractions contenant la substance de Rf=0,5 sont réunies et concentrées pour obtenir le composé **38** (0,01 g, 6%).
SM (CI, Isobut.) : m/z 296 (M+1). HRMS calculé pour C₁₉H₂₂NO₂: 296,1650. On a trouvé 296,1658.

### Procédé général pour la préparation des 3-benzyloxycarbonylamino-1,4-butyrolactones

Une solution de lactones (R,S)-**14** ou **15** (1,63 mmol) dans de l'éthanol (10 ml) contenant du palladium sur du carbone (10%) est agité sous hydrogène (40 p.s.i.) pendant 16 heures. Le mélange est filtré, le solvant du filtrat est évaporé et le résidu est utilisé dans l'étape suivante sans autre purification. Au mélange résultant contenant les 4-aminolactones, du CH₂Cl₂ (3 ml), de l'Et₃N (0,4 ml) et du DMAP (0,045g) est ajouté du chloroformate de benzyle (1,2 equiv) à 0°C et le mélange réactionnel est agité à cette température pendant 30 minutes. Après 12 heures à température ambiante le solvant est enlevé sous pression réduite et le résidu est purifié par chromatographie flash.

### Les cis et trans-3-Benzyloxycarbonylamino-2-C-méthyl-1,4-butyrolactones

***27*** *et* ***28*** sont préparées comme décrit ci-dessus à partir du composé **14** (R₁= CH₃). Les produits sont chromatographiés sur gel de silice en utilisant EtOAc/hexanes (1/1) comme éluant. Les fractions contenant la substance de Rf=0,4 sont réunies et concentrées pour obtenir le composé **28** (27%) dont le point de fusion est 127-128°C.
SM (CI, Isobut.) : m/z 250 (M+1). HRMS calculé pour C₁₃H₁₅NO₄ : 249,1000. On a trouvé 249,0978.

Les fractions contenant la substance de Rf=0,5 sont réunies et concentrées pour obtenir le composé **27** (15%).
SM (FAB, Thioglycérol) : m/z 248 (M-1). HRMS calculé pour C₁₃H₁₅NO₄: 249,1000. On a trouvé 249,0992.

*La 3-Benzyloxycarbonylamino-2,2-C-dipropyl-1,4-butyrolactone* ***30*** est préparée comme décrit ci-dessus à partir du composé 16. Le produit est chromatographié sur gel de silice en utilisant EtOAc/hexanes (1/1) comme éluant. Les fractions contenant la substance de Rf=0,7 sont réunies et concentrées pour obtenir le composé **30** sous forme d'un solide blanc (52%) ayant un point de fusion de 91-92°C.
SM (CI, Isobut.): m/z 320 (M+1). Infrarouge (KBr) : 1556,7, 1684-1698 (O-CO-N), 1774,1 (CO lactone) cm⁻¹. Analyse élémentaire pour C₁₈H₂₅NO₄ : Calculée, % : C, 67,69; H, 7,89; N, 4,39. Trouvée, % : C, 67,94; H, 7,84; N, 4,25.

*La 3-Benzyloxycarbonylamino-1,4-butyrolactone* ***26*** est préparée comme décrit ci-dessus à partir du composé **13.** Le produit est chromatographié sur gel de silice en utilisant EtOAc/hexanes (1/1) comme éluant. Les fractions contenant la substance de Rf=0,4 sont réunies et concentrées pour obtenir le composé **26** sous forme d'un solide blanc (61%) ayant un point de fusion de 101-102°C.
SM (CI, Isobut.) : m/z 236 (M+1). Infrarouge (KBr) : 1552, 1673-1693 (O-CO-N), 1779,4 (CO lactone), 3307,7 (NH) cm⁻¹. Analyse élémentaire pour C₁₂H₁₃NO₄ : Calculée, % : C,61,27; H, 5,57; N, 5,95. Trouvée, % : C 61,39; H, 5,66; N, 5,91.

### Procédé général pour la préparation des 3-(N-benzyl,N-benzyloxycarbonyl)amino-1,4-butyrolactones

A un mélange de 3-benzylamino-2-C-méthyl-1,4-lactone **14** (1,63 mmol), d'Et₃N (0,4 ml) et de DMAP (0,045 g) dans du CH₂Cl₂ (5 ml) est ajouté du chloroformate de benzyle (1,2 equiv) à 0°C. Après 30 minutes à 0°C puis 12 heures à température ambiante, le solvant est éliminé sous pression réduite et le résidu est purifié par chromatographie flash.

*Les cis et trans-3-(N-benzyl,N-benzyloxycarbonyl)amino-2-C-méthyl-1,4-butyrolactones* ***43*** *et* ***42*** sont préparés comme décrit ci-dessus à partir du composé **14** (R₁= CH₃). Les produits sont chromatographiés sur gel de silice en utilisant EtOAc/hexanes (1/1) comme éluant. Les fractions contenant la substance de Rf=0,6 sont réunies et concentrées pour obtenir le composé **43** (11%).
Analyse élémentaire pour C₂₀H₂₁NO₄ : Calculée, % : C, 68,55; H, 6,71. Trouvée, % : C, 68,18; H, 6,56.

Les fractions contenant la substance de Rf=0,7 sont réunies et concentrées pour obtenir le composé **42** (22%).
Analyse élémentaire pour C₂₀H₂₁NO₄ : Calculée, % : C, 68,55; H, 6,71.Trouvée, % : C, 68,42; H, 6,38.

*L'anhydride du N-(benzyloxycarbonyl)-aspartique D (****25****)* est préparé à partir de l'acide N-(carbobenzyloxy)-aspartique D (**24**) par la méthode de McGarvey et al. (G.J. McGarvey, et al., J. Amer. Chem. Soc., 1986, 108, 4943) avec un rendement global de 80% par rapport à l'acide aspartique D (D-**23**). Le point de fusion est de 126-127°C. [α]_{D} +26° (c 1,22, MeOH). Infrarouge (KBr) : 1529, 1697 (O-CO-N), 1781 et 1866 (anhydride).

*La 3-(R)-[benzyloxycarbonyl)amino]-1,4-butyrolactone (R)-****26***
A une bouillie agitée de 0,138 g (3,6 mmol) de borohydrure de sodium dans du THF (7 ml) à 0°C est ajoutée goutte à goutte 0,9 g (3,6 mmol) d'anhydride **25** dans du THF (8 ml). Après agitation à température ambiante pendant 1 heure, le mélange réactionnel est acidifié prudemment jusqu'à pH 2 avec 6N d'HCl et est alors concentré jusqu'à approximativement un quart de son volume sous pression réduite (aspiration d'eau). Le résidu est dilué avec de l'eau et extrait avec 4 portions d'acétate d'éthyle puis les extraits organiques réunis sont concentrés sous pression réduite. Le produit brut est purifié par chromatographie flash (1/1 : EtOAc/hexanes). Les fractions contenant la substance de Rf=0,4 sont réunies et concentrées pour obtenir le composé *(R)*-**26** sous forme d'un solide blanc (50%) ayant un point de fusion de 101-102°C.
[α]_{D} + 49,2° (c 1, CHCl₃). SM (CI, Isobut.) : m/z 236 (M+1). Infrarouge (KBr) : 1558, 1674-1694 (O-CO-N), 1780 (CO lactone), 3307,4 (NH) cm⁻¹.

*La 3-(R)-benzylamino-1,4-butyrolactone (R)-****13***
Une solution de 0,15 g de lactone *(R)*-**26** (0,64 mmol) dans de l'EtOAc (10 ml) contenant du palladium sur du carbone (10%) est agitée sous hydrogène (30 p.s.i.) à température ambiante pendant 4 heures. Le mélange est filtré, le solvant du filtrat est évaporé et le résidu est utilisé dans l'étape suivante sans autre purification. Au mélange résultant contenant la 3-aminolactone dans du méthanol (3 ml), 0,065 ml (0,64 mmol) de benzaldéhyde est ajouté avec refroidissement. Après avoir laissé la solution au repos pendant 1 heure, elle est diluée avec du méthanol pour obtenir un volume total de 20 ml et hydrogénée avec un catalyseur d'oxyde de platine sous une pression de 40 p.s.i.. La réduction est terminée en trois heures. Après avoir ôté le catalyseur, le solvant est évaporé et le résidu est purifié par chromatographie flash (1/1 : EtOAc/hexanes). Les fractions contenant la substance de Rf=0,07 sont réunies et concentrées pour obtenir le composé (*R*)-**13** sous forme d'une huile incolore (0,07 g, 57%).
[α]_{D} + 16,5° (c 1, CHCl₃). RMN H¹ (200 MHz, CDCl₃) δ : 1,62 (1H, s, NH), 2,38 (1H, dd, J₁=17,5 Hz, J₂=4,6 Hz), 2,70 (1H, dd, J₁, J₃=7,1Hz), 3,67 (1H, m), 3,79 (2H, s), 4,11 (1H, dd, J₄=9,5 Hz, J₅=3,9 Hz), 4,36 (1H, dd, J₄, J₅=5,9 Hz), 7,26-7,37 (5H, m).

*La 3-(R)-benzylamino-2,2-di-C-allyl-1,4-butyrolactone (R-****16***) est synthétisée de la façon décrite précédemment pour le composé *R,S*-**16**. Brièvement, une solution d'énolate de lithium préparée à partir de la lactone (*R*)-**13** (0,092g, 0,488 mmol) à -78°C est traitée avec une solution de bromure d'allyle (5 equiv, 0,23 ml, 2,44 mmol) dans du HMPA (1 ml). Le mélange résultant est agité à -78°C pendant 2 heures. La réaction est alors neutralisée à 0°C pour obtenir une huile jaune. Le produit brut est purifié par chromatographie flash (1/1: EtOAc/hexanes). Les fractions contenant la substance de Rf=0,4 sont réunies et concentrées pour obtenir le dérivé mono-allyle (0,01 g, 9%).
[α]_{D} + 32,6° (c 1, CHCl₃). RMN H¹ (300 MHz, CDCl₃) δ : 1,64 (1H, NH), 2,24 (1H, m), 2,39 (2H, m), 2,60 (1H, m), 3,75 (2H, q, J₁=12,1 Hz), 4,23 (1H, dd, J₂=9,03 Hz, J₃=6,41 Hz), 4,36 (1H, dd, J₂, J₄=6,64 Hz), 5,24 (2H, m), 5,85 (1H, m), 7,31-7,35 (5H, m).

Les fractions contenant la substance de Rf=0,7 sont réunies et concentrées pour obtenir le composé diallyle *R*-**16** (0,068g, 52%).
[α]_{D} +1,4° (c 0,8, CHCl₃). RMN H¹ (300 MHz, CDCl₃) δ : 1,51 (1H, NH), 2,32 (2H, m), 2,47 (2H, m), 3,54 (1H, dd, J₁=8,43 Hz, J2=7,68 Hz), 3,80 (2H, s), 3,81 (1H, dd, J₃=17,55 Hz, J₄= 6,4 Hz), 4,28 (1H, dd, J3, J₅=7,68 Hz), 5,00-5,22 (4H, m), 5,68 (1H, m), 5,88 (1H, m), 7,30-7,36 (5H, m).

*La 3-(R)-Benzyloxycarbonylamino-3,3-di-C-propyl-1,4-butyrolactone (R*-***30****)* est préparée à partir du composé *R*-**16** comme décrit ci-dessus pour le composé *R,S*-**30**. Le produit est chromatographié sur gel de silice en utilisant EtOAc/hexanes (1/1) comme éluant. Les fractions contenant la substance de Rf=0,7 sont réunies et concentrées pour obtenir le composé *R*-**30** sous forme d'une huile incolore (57%). [α]_{D} -0,28° (c 1,44, CHCl₃). RMN H¹ (250 MHz, CDCl₃) δ : 0,90 (3H, t, J₁=7,2 Hz), 0,92 (3H, t, J₂=7,1 Hz), 1,18-1,69 (8H, m), 3,84 (1H, dd, J₃=8,6 Hz, J₄=7,4 Hz), 4,45-4,58 (2H, m), 4,88 (1H, NH, d, J₅=8,28 Hz), 5,12 (2H, s), 7,36 (5H, s).
Ce produit est identique par analyse HPLC (temps de rétention : 12,3 min) à la substance obtenue à partir des séparations par HPLC analytiques du mélange racémique (éluant 9/1: hexanes/isopropanol) sur une colonne OD chirale.

### Procédé général pour la préparation des 3-sulfonyl- et acylamino-2,2-di-C-propyl-1,4-butyrolactones 31-36

Une solution de lactone *R,S-***16** (1,63 mmol) dans de l'éthanol (10 ml) contenant du palladium sur du carbone (10%) est agitée sous hydrogène (40 p.s.i.) pendant 16 heures. Le mélange est filtré, le solvant du filtrat est évaporé et le résidu est utilisé dans l'étape suivante sans autre purification. Au mélange résultant contenant la 3-amino-2,2-dipropyl-butyrolactone, du CH₂Cl₂ (3 ml), de l'Et₃N (0,4 ml) et du DMAP (0,045 g), est ajouté R-C1 (1,2 equiv) à 0°C et le mélange réactionnel est agité à cette température pendant 30 minutes. Après 12 heures à température ambiante le solvant est éliminé sous pression réduite et le résidu est purifié par chromatographie flash.

*La 3-(p-chlorophénylsulfonamido)-2,2-di-C-propyl-1,4-butyrolactone* ***31*** est préparée à partir du composé **16** et du chlorure de p-chlorophénylsulfonyle de la façon décrite ci-dessus. Le produit est chromatographié sur gel de silice en utilisant EtOAc/hexanes (1/1) comme éluant. Les fractions contenant la substance de Rf=0,7 sont réunies et concentrées pour obtenir le composé **31** sous forme d'un solide blanc (84%) ayant un point de fusion de 96-97°C.
SM (CI, Isobut.) : m/z 360 (M+1). Infrarouge (KBr) : 1173,7 et 1477,7 (SO₂N-), 1757,7 (CO lactone) cm⁻¹. Analyse élémentaire pour C₁₆H₂₂NO₄SCl+0,1 hexane : Calculée, % : C, 54,11; H, 6,40; N, 3,80; S, 8,70. Trouvée, % : C, 54,22; H, 6,19; N, 3,57; S, 8,65.

*La 3-(p-trifluorométhylphénylsulfonamido)-2,2-di-C-propyl-1,4-butyrolactone* ***32*** est préparée de la façon décrite ci-dessus à partir du composé **16** et du chlorure de p-trifluoro méthylphénylsulfonyle. Le produit est chromatographié sur gel de silice en utilisant EtOAc/hexanes (1/1) comme éluant. Les fractions contenant la substance de Rf=0,6 sont réunies et concentrées pour obtenir le composé 32 sous forme d'un solide blanc (57 %) ayant un point de fusion de 106-107°C.
SM (CI, Isobut.) : m/z 394 (M+1). Infrarouge (KBr) : 1168,9 et 1323,3 (SO₂N-), 1752,9 (CO lactone) cm⁻¹. Analyse élémentaire pour C₁₇H₂₂NO₄SF₃+0,1 H2O : Calculée, % : C, 51,66; H, 5,66; N, 3,54; S, 8,11. Trouvée, % : C, 51,27 ; H, 5,31; N, 3,32 ; S, 8,42.

*La 3-(p-méthoxyphénylsulfonamido)-2,2-di-C-propyl-1,4-butyrolactone* ***33*** est préparée de la façon décrite ci-dessus à partir du composé **16** et du chlorure de p-méthoxyphénylsulfonyle. Le produit est chromatographié sur gel de silice en utilisant EtOAc/hexanes (1/1) comme éluant. Les fractions contenant la substance de Rf=0,6 sont réunies et concentrées pour obtenir le composé **33** sous forme d'un solide blanc (84 %) ayant un point de fusion de 103-104°C.
SM (CI, Isobut.) : m/z 356 (M+1). Infrarouge (KBr) : 1167,8 et 1323,3 (SO₂N-), 1753,7 (CO lactone) cm⁻¹. Analyse élémentaire pour C₁₇H₂₅NO₅S+0,1 hexane : Calculée, % : C, 58,06; H, 7,31; N, 3,85; S, 8,81. Trouvée, % : C, 58,28; H, 7,21; N, 3,96; S, 8,46.

*La 3-benzoylamino-2,2-di-C-propyl-1,4-butyrolactone* ***34*** est préparée de la façon décrite ci-dessus à partir du composé **16** et du chlorure de benzoyle. Le produit est chromatographié sur gel de silice en utilisant EtOAc/hexanes (1/1) comme éluant. Les fractions contenant la substance de Rf=0,5 sont réunies et concentrées pour obtenir le composé **34** sous forme d'un solide blanc (57 %) ayant un point de fusion de 130-131°C.
SM (CI, Isobut.) : m/z 290 (M+1). Infrarouge (KBr) : 1546,8, 1637,1 (CO-N), 1766,69 (CO lactone), 3306,4 (NH) cm⁻¹. Analyse élémentaire pour C₁₇H₂₃NO₃ : Calculée, % : C, 70,56; H, 8,01; N, 4,84. Trouvée, % : C, 70,37; H, 7,91; N, 4,75.

*La 3-allyloxycarbonylamino-2,2-di-C-propyl-1,4-butyrolactone* ***35*** est préparée de la façon décrite ci-dessus à partir du composé **16** et du chlorure d'allyloxycarbonyle. Le produit est chromatographié sur gel de silice en utilisant EtOAc/heptanes (1/1) comme éluant. Les fractions contenant la substance de Rf=0,6 sont réunies et concentrées pour obtenir le composé 35 sous forme d'un solide blanc (81 %) ayant un point de fusion de 80-81°C.
SM (CI, Isobut.): m/z 270 (M+1). Infrarouge (KBr) : 1557,2, 1686,1 (O-CO-N), 1777,4 (CO lactone), 3315,9 (NH) cm⁻¹. Analyse élémentaire pour C₁₄H₂₃NO₄ : Calculée, % : C, 62,43; H, 8,61; N, 5,20. Trouvée, % : C, 62,41; H, 8,49 ; N,5,09.

*La 3-éthyloxycarbonylamino-2,2-di-C-propyl-1,4-butyrolactone* ***36*** est préparée de la façon décrite ci-dessus à partir du composé **16** et du chlorure d'éthyloxycarbonyle. Le produit est chromatographié sur gel de silice en utilisant EtOAc/heptanes (1/1) comme éluant. Les fractions contenant la substance de Rf=0,5 sont réunies et concentrées pour obtenir le composé **36** sous forme d'un solide blanc (77 %) ayant un point de fusion de 111-112°C.
SM (CI, Isobut.) : m/z 258 (M+1). Infrarouge (KBr) : 1548,39, 1690,1 (O-CO-N), 1787,8 (CO lactone), 3326,2 (NH) cm⁻¹. Analyse élémentaire pour C₁₃H₂₃NO₄ : Calculée, % : C, 60,68; H, 9,01; N, 5,44. Trouvée, % : C, 60,67; H, 8,86; N, 5,43.

*La 3-tert-butyloxycarbonylamino-2,2-di-C-propyl-1,4-butyrolactone* ***37*** est préparée de la façon décrite ci-dessus à partir du composé **16** et du dicarbonate de di-tert-butyle. Le produit est chromatographié sur gel de silice en utilisant EtOAc/heptanes (1/1) comme éluant. Les fractions contenant la substance de Rf=0,7 sont réunies et concentrées pour obtenir le composé **37** sous forme d'un solide blanc (70%) ayant un point de fusion de 134-135°C.
SM (EI, MeOH) : m/z 285 (M). Infrarouge (KBr) : 1683,3 (O-CO-N), 1768,6 (CO lactone), 3340,2 (NH) cm⁻¹. Analyse élémentaire pour C₁₅H₂₇NO₄ : Calculée, % : C, 63,13; H, 9,54; N, 4,91. Trouvée, % : C, 63,33; H, 9,48; N, 4,89.

*La 3-[p-aminophénylsulfonamido]-2,2-di-C-propyl-1,4-butyrolactone* ***44*** est préparée de la façon décrite ci-dessus à partir du composé **16** et du chlorure de p-nitrophénylsulfonyle. Le produit est chromatographié sur gel de silice en utilisant EtOAc/heptanes (1/1) comme éluant. Les fractions contenant la substance de Rf=0,6 sont réunies et concentrées pour obtenir la 3-[p-nitro phénylsulfonamido]-2,2-di-C-propyl-1,4-butyrolactone (94%). Cette dernière est réduite par hydrogénation catalytique de la façon décrite ci-dessus pour obtenir le composé **44** (Rf=0,3, 87%).
SM (EI, MeOH) : m/z 340 (M). Analyse élémentaire pour C₁₆H₂₄N₂O₄S + 0,5 H₂O : Calculée, % : C, 54,99; H, 7,21; N, 7,02; S, 9,17. Trouvée, % : C, 55,11; H, 6,88 ; N, 6,96 ; S, 9,03.

*La 3-[p-(allyloxycarbonylamino)phénylsulfonamido]-2,2-di-C-propyl-1,4-butyrolactone* ***45*** est préparée de la façon décrite ci-dessus à partir du composé **44** et du chloroformate d'allyle. Le produit est chromatographié sur gel de silice en utilisant EtOAc/heptanes (1/1) comme éluant. Les fractions contenant la substance de Rf=0,5 sont réunies et concentrées pour obtenir le composé hygroscopique **45** (77%).
SM (EI, MeOH) : m/z 424 (M). Analyse élémentaire pour C₂₀H₂₈N₂O₆S + 0,1 C₇H₁₆ : Calculée, % : C, 57,22 ; H, 6,87; N, 6,15. Trouvée, % : C, 57,08; H, 6,81; N, 5,94.

## Revendications

1. Composé représenté par la formule générale suivante : 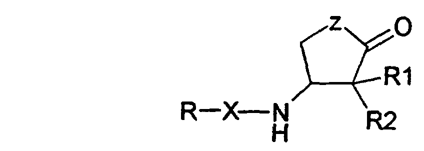 dans laquelle :
Z représente un atome de soufre ou d'oxygène,
les groupes R1 et R2, qui peuvent être identiques ou différents l'un de l'autre, représentent chacun un groupe alkyle ou un groupe alcényle
X représente un CO, un CO₂, un SO ou un SO₂
et le groupe R représente un groupe alkyle, aryle, alcényle ou aralkyle à la condition que lorsque Z représente un atome d'oxygène, X un SO₂ et R un groupe 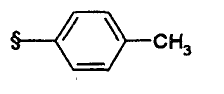
R1 et R2 ne représentent pas tous les deux le groupe méthyl.

2. Composé selon la revendication 1 **caractérisé en ce que** Z représente un atome d'oxygène.

3. Composé selon la revendication 2 **caractérisé en ce qu'**il est représenté par la formule générale : 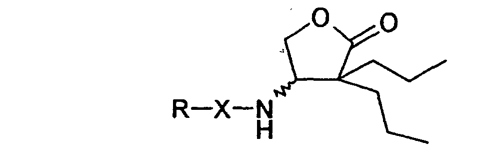 dans laquelle :
X représente un CO, un CO₂, un SO ou un SO₂
et le groupe R représente un groupe alkyle, aryle, alcényle ou aralkyle.

4. Composé selon les revendications 1 à 3 **caractérisé en ce qu'**il est représenté par la formule :

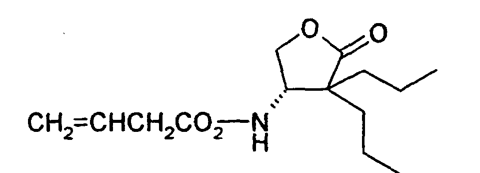

5. Procédé de préparation des composé de formule générale **I** suivante : 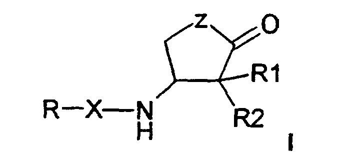 dans laquelle :
Z représente un atome de soufre ou d'oxygène,
les groupes R1 et R2, qui peuvent être identiques ou différents l'un de l'autre, représentent chacun un groupe alkyle ou un groupe alcényle
X représente un CO, un CO₂, un SO ou un SO₂
et le groupe R représente un groupe alkyle, aryle, alcényle ou aralkyle
**caractérisé en ce que** l'amine du composé de formule générale : 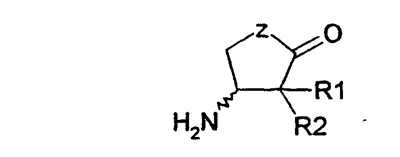 dans laquelle :
Z représente un atome de soufre ou d'oxygène,
et les groupes R1 et R2, qui peuvent être identiques ou différents l'un de l'autre, représentent chacun un groupe alkyle ou un groupe alcényle
est traité par un réactif approprié pour obtenir les composés de formule générale I.

6. Procédé de préparation selon la revendication 5 **caractérisé en ce qu'**il comporte une étape préalable d'hydrogénolyse pour enlever le groupement benzyle du composé de formule générale : 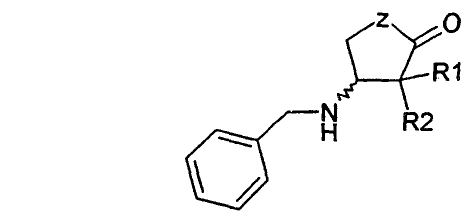 dans laquelle :
Z représente un atome de soufre ou d'oxygène,
et les groupes R1 et R2, qui peuvent être identiques ou différents l'un de l'autre, représentent chacun un groupe alkyle ou un groupe alcényle

7. Procédé de préparation selon la revendication 6 **caractérisé en ce qu'**il comporte une étape préalable d'alkylation en position C-2 du composé de formule générale : 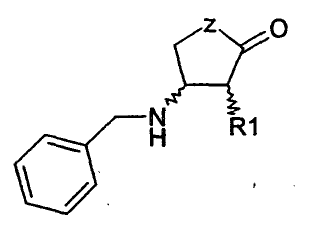 dans laquelle :
Z représente un atome de soufre ou d'oxygène,
et le groupe R1 représente un groupe alkyle ou un groupe alcényle
par traitement avec une base forte suivie de l'addition d'un agent alkylant de formule générale R2X dans laquelle R2 représente un groupe alkyle, alcényle ou aralkyle.

8. Procédé de préparation selon la revendication 7 **caractérisé en ce qu'**il comporte une étape préalable de monoalkylation en position C-2 de la 3-benzylaminolactone de formule générale : 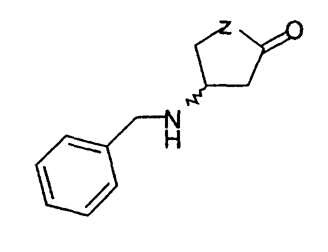 dans laquelle :
Z représente un atome de soufre ou d'oxygène,
par traitement avec une base forte suivie de l'addition d'un agent alkylant de formule générale R1X dans laquelle R1 représente un groupe alkyle, alcényle ou aralkyle.

9. Procédé de préparation selon la revendication 8 **caractérisé en ce qu'**il comporte une étape préalable d'addition 1, 4 de type Michaël de la benzylamine sur une butyrolactone-1, 4 ou une thiobutyrolactone-1, 4 insaturée pour donner la 3-benzylaminolactone correspondante.

10. Procédé de préparation selon la revendications 9 **caractérisé en ce que** le produit de départ de la première étape est un acide aspartique D ou L et les composés selon les revendication 1 à 3 obtenus sont optiquement purs.

11. Procédé de préparation selon les revendications 7 à 10 **caractérisé en ce que** la base forte utilisée pour réaliser les alkylations est l'hexaméthyldisilazide de lithium.

12. Procédé de préparation selon les revendications 5 à 11 **caractérisé en ce que** le réactif permettant de traiter l'amine pour obtenir les composé selon les revendications 1 à 4 fait partie de la famille des chlorures d'acyle, chlorures de sulfonyle ou chloroformiates.

13. Composition pharmaceutique comprenant un composé de formule générale suivante : 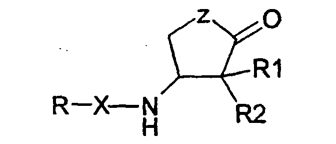 dans laquelle :
Z représente un atome de soufre ou d'oxygène,
les groupes R1 et R2, qui peuvent être identiques ou différents l'un de l'autre, représentent chacun un groupe alkyle ou un groupe alcényle
X représente un CO, un CO₂, un SO ou un SO₂
et le groupe R représente un groupe alkyle, aryle, alcényle ou aralkyle
et un support pharmaceutique approprié.

14. Utilisation des composés de formule générale suivante : 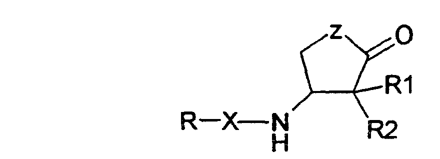 dans laquelle :
Z représente un atome de soufre ou d'oxygène,
les groupes R1 et R2, qui peuvent être identiques ou différents l'un de l'autre, représentent chacun un groupe alkyle ou un groupe alcényle
X représente un CO, un CO₂, un SO ou un SO₂
et le groupe R représente un groupe alkyle, aryle, alcényle ou aralkyle
et d'une composition selon la revendication 13 pour la préparation d'un stimulant de l'activité de l'acide γ-aminobutyrique agissant via le récepteur GABA-A du système nerveux central.

15. Composé de formule générale suivante : 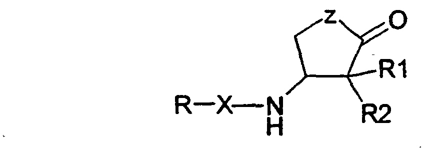 dans laquelle :
Z représente un atome de soufre ou d'oxygène,
les groupes R1 et R2, qui peuvent être identiques ou différents l'un de l'autre, représentent chacun un groupe alkyle ou un groupe alcényle
X représente un CO, un CO₂, un SO ou un SO₂
et le groupe R représente un groupe alkyle, aryle, alcényle ou aralkyle
et composition selon la revendication 13 pour son utilisation comme médicament.

16. Utilisation des composés de formule générale suivante : 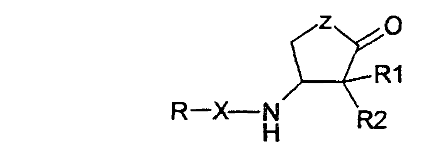 dans laquelle :
Z représente un atome de soufre ou d'oxygène,
les groupes R1 et R2, qui peuvent être identiques ou différents l'un de l'autre, représentent chacun un groupe alkyle ou un groupe alcényle
X représente un CO, un CO₂, un SO ou un SO₂
et le groupe R représente un groupe alkyle, aryle, alcényle ou aralkyle
et d'une composition selon la revendication 13 pour la fabrication d'un médicament destiné au traitement des troubles nerveux.

17. Utilisation selon la revendication 16 **caractérisée en ce que** les troubles nerveux sont du type épilepsie, anxiété, dépression, troubles du sommeil, attaques de panique, contractions musculaires, douleur, dépendance à l'alcool ou aux benzodiazépines ou comportements psychotiques.

## Claims

1. Compound represented by the following general formula: 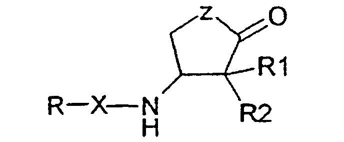 in which:
Z represents a sulphur or oxygen atom,
the R1 and R2 groups, which can be identical to or different from one another, each represent an alkyl group or an alkenyl group,
X represents a CO, a CO₂, an SO or an SO₂,
and the R group represents an alkyl, aryl, alkenyl or aralkyl group,
provided that, when Z represents an oxygen atom, X an SO₂ and R a 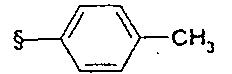 group,
R1 and R2 do not both represent the methyl group.

2. Compound according to Claim 1, **characterized in that** Z represents an oxygen atom.

3. Compound according to Claim 2, **characterized in that** it is represented by the general formula: 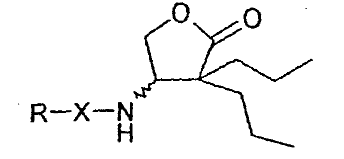 in which:
X represents a CO, a CO₂, an SO or an SO₂,
and the R group represents an alkyl, aryl, alkenyl or aralkyl group.

4. Compound according to Claims 1 to 3, **characterized in that** it is represented by the formula:

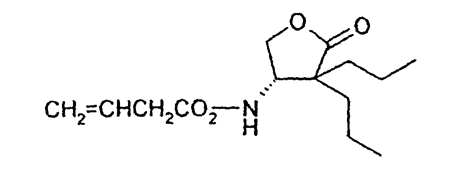

5. Process for the preparation of the compound of following general formula I: 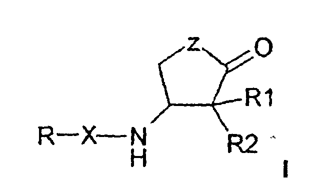 in which:
Z represents a sulphur or oxygen atom,
the R1 and R2 groups, which can be identical to or different from one another, each represent an alkyl group or an alkenyl group,
X represents a CO, a CO₂, an SO or an SO₂,
and the R group represents an alkyl, aryl, alkenyl or aralkyl group,
**characterized in that** the compound of general formula: 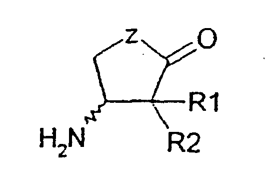 in which:
Z represents a sulphur or oxygen atom,
and the R1 and R2 groups, which can be identical to or different from one another, each represent an alkyl group or an alkenyl group,
is treated with an appropriate reactant to produce the compound of general formula I.

6. Preparation process according to Claim 5, **characterized in that** it comprises a preliminary hydrogenolysis stage to remove the benzyl group from the compound of general formula: 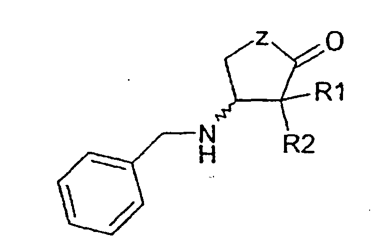 in which:
Z represents a sulphur or oxygen atom,
and the R1 and R2 groups, which can be identical to or different from one another, each represent an alkyl group or an alkenyl group.

7. Preparation process according to Claim 6, **characterized in that** it comprises a preliminary stage of alkylation in the C-2 position of the compound of . general formula: 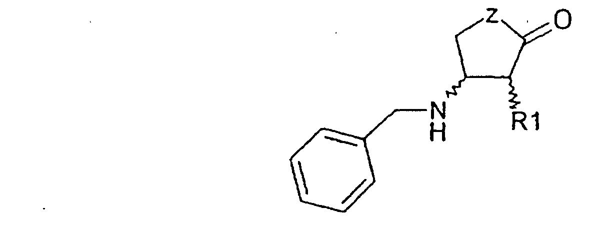 in which:
Z represents a sulphur or oxygen atom,
and the R1 group represents an alkyl group or an alkenyl group,
by treatment with a strong base, followed by the addition of an alkylating agent of general formula R2X in which R2 represents an alkyl, alkenyl or aralkyl group.

8. Preparation process according to Claim 7, **characterized in that** it comprises a preliminary stage of monoalkylation in the C-2 position of the 3-benzylaminolactone of general formula: 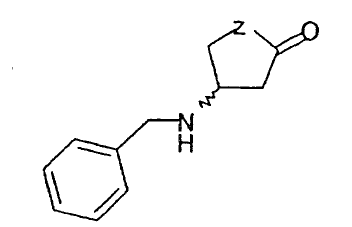 in which:
Z represents a sulphur or oxygen atom,
by treatment with a strong base, followed by the addition of an alkylating agent of general formula R1X in which R1 represents an alkyl, alkenyl or aralkyl group.

9. Preparation process according to Claim 8, **characterized in that** it comprises a preliminary stage of 1,4 addition of Michael type of benzylamine to an unsaturated 1,4-butyrolactone or 1,4-thiobutyrolactone to give the corresponding 3-benzylaminolactone.

10. Preparation process according to Claim 9, **characterized in that** the starting material in the first stage is a D- or L-aspartic acid and the compounds according to Claims 1 to 3 obtained are optically pure.

11. Preparation process according to Claims 7 to 10, **characterized in that** the strong base used to carry out the alkylations is lithium hexamethyldisilazide.

12. Preparation process according to Claims 5 to 11, **characterized in that** the reactant which makes it possible to treat the amine in order to obtain the compounds according to Claims 1 to 4 is one of the family of the acyl chlorides, sulphonyl chlorides or chloroformates.

13. Pharmaceutical composition comprising a compound of following general formula: 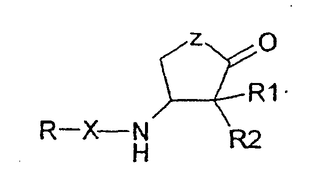 in which:
Z represents a sulphur or oxygen atom,
the R1 and R2 groups, which can be identical to or different from one another, each represent an alkyl group or an alkenyl group,
X represents a CO, a CO₂, an SO or an SO₂,
and the R group represents an alkyl, aryl, alkenyl or aralkyl group,
and an appropriate pharmaceutical carrier.

14. Use of the compound of following general formula: 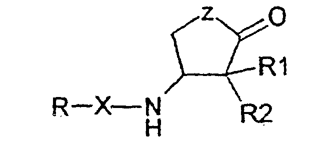 in which:
Z represents a sulphur or oxygen atom,
the R1 and R2 groups, which can be identical to or different from one another, each represent an alkyl group or an alkenyl group,
X represents a CO, a CO₂, an SO or an SO₂,
and the R group represents an alkyl, aryl, alkenyl or aralkyl group,
and of a composition according to Claim 13 for the preparation of a stimulant of the activity of γ-aminobutyric acid acting via the GABA-A receptor of the central nervous system.

15. Compound of following general formula: 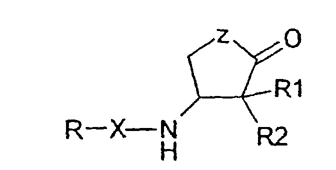 in which:
Z represents a sulphur or oxygen atom,
the R1 and R2 groups, which can be identical to or different from one another, each represent an alkyl group or an alkenyl group,
X represents a CO, a CO₂, an SO or an SO₂,
and the R group represents an alkyl, aryl, alkenyl or aralkyl group,
and a composition according to Claim 13 for its use as medicament.

16. Use of the compound of following general formula: 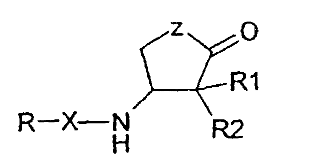 in which:
Z represents a sulphur or oxygen atom,
the R1 and R2 groups, which can be identical to or different from one another, each represent an alkyl group or an alkenyl group,
X represents a CO, a CO₂, an SO or an SO₂,
and the R group represents an alkyl, aryl, alkenyl or aralkyl group,
and of a composition according to Claim 13 for the manufacture of a medicament intended for the treatment of nervous disorders.

17. Use according to Claim 16, **characterized in that** the nervous disorders are of the following types: epilepsy, anxiety, depression, sleep disorders, panic attacks, muscular contractions, pain, dependence on alcohol or benzodiazepines, or psychotic behaviours.

## Patentansprüche

1. Verbindung, welche durch die folgende allgemeine Formel angegeben wird: 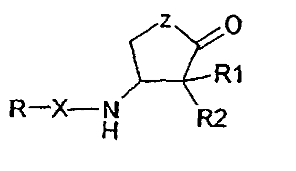 in welcher:
Z für ein Schwefel- oder Sauerstoffatom steht,
die Gruppen R1 und R2, die gleich oder voneinander verschieden sein können, jeweils für eine Alkylgruppe oder eine Alkenylgruppe stehen,
X für ein CO, ein CO₂, ein SO oder ein SO₂ steht
und die Gruppe R für eine Alkyl-, Aryl-, Alkenyl- oder Arylalkylgruppe steht,
mit der Maßgabe, dass, wenn Z für ein Sauerstoffatom steht, X für ein SO₂ steht und R für eine Gruppe 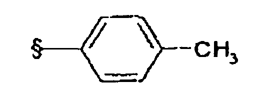 steht,
R1 und R2 nicht alle beide für die Methylgruppe stehen.

2. Verbindung nach Anspruch 1, **dadurch gekennzeichnet, dass** Z für ein Sauerstoffatom steht.

3. Verbindung nach Anspruch 2, **dadurch gekennzeichnet, dass** sie angegeben wird durch die allgemeine Formel: 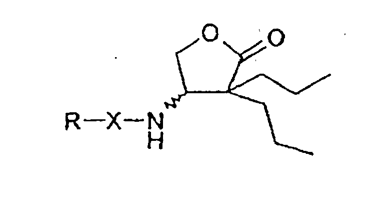 in welcher:
X für ein CO, ein CO₂, ein SO oder ein SO₂ steht
und die Gruppe R für eine Alkyl-, Aryl-, Alkenyl- oder Arylalkylgruppe steht.

4. Verbindung nach den Ansprüchen 1 bis 3, **dadurch gekennzeichnet, dass** sie angegeben wird durch die Formel:

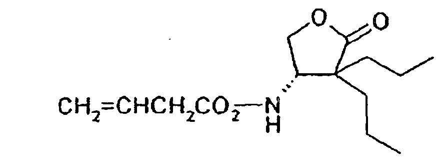

5. Verfahren zur Herstellung der Verbindungen der folgenden allgemeinen Formel **I**: 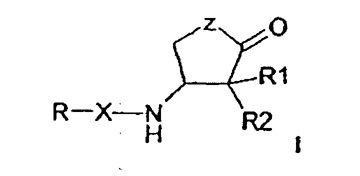 in welcher:
Z für ein Schwefel- oder Sauerstoffatom steht,
die Gruppen R1 und R2, die gleich oder voneinander verschieden sein können, jeweils für eine Alkylgruppe oder eine Alkenylgruppe stehen,
X für ein CO, ein CO₂, ein SO oder ein SO₂ steht
und die Gruppe R für eine Alkyl-, Aryl-, Alkenyl- oder Arylalkylgruppe steht,
**dadurch gekennzeichnet, dass** das Amin der Verbindung der allgemeinen Formel: 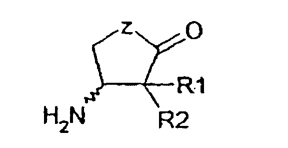 in welcher:
Z für ein Schwefel- oder Sauerstoffatom steht
und die Gruppen R1 und R2, die gleich oder voneinander verschieden sein können, jeweils für eine Alkylgruppe oder eine Alkenylgruppe stehen,
mit einem geeigneten Reagens behandelt wird, um die Verbindungen der allgemeinen Formel I zu erhalten.

6. Herstellungsverfahren nach Anspruch 5, **dadurch gekennzeichnet, dass** es einen vorab erfolgenden Schritt einer Hydrogenolyse umfasst, um die Benzylgruppe der Verbindung der allgemeinen Formel: 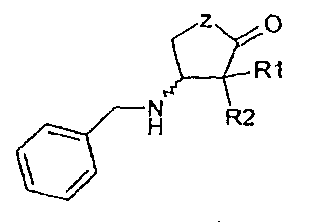 in welcher:
Z für ein Schwefel- oder Sauerstoffatom steht und die Gruppen R1 und R2, die gleich oder voneinander verschieden sein können, jeweils für eine Alkylgruppe oder eine Alkenylgruppe stehen,
zu entfernen.

7. Herstellungsverfahren nach Anspruch 6, **dadurch gekennzeichnet, dass** es einen vorab erfolgenden Schritt einer Alkylierung der Verbindung der allgemeinen Formel: 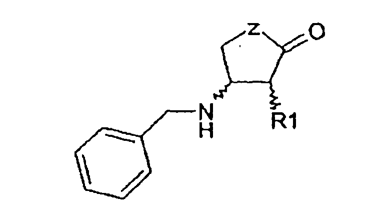 in welcher:
Z für ein Schwefel- oder Sauerstoffatom steht
und die Gruppe R1 für eine Alkylgruppe oder eine Alkenylgruppe steht,
an Position C-2 durch Behandlung mit einer starken Base, gefolgt von der Zugabe eines Alkylierungsmittels der allgemeinen Formel R2X, in welcher R2 für eine Alkyl-, Alkenyl- oder Arylalkylgruppe steht, umfasst.

8. Herstellungsverfahren nach Anspruch 7, **dadurch gekennzeichnet, dass** es einen vorab erfolgenden Schritt einer Monoalkylierung des 3-Benzylaminolactons der allgemeinen Formel: 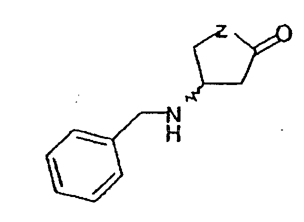 in welcher:
Z für ein Schwefel- oder Sauerstoffatom steht,
an Position C-2 durch Behandlung mit einer starken Base, gefolgt von der Zugabe eines Alkylierungsmittels der allgemeinen Formel R1X, in welcher R1 für eine Alkyl-, Alkenyl- oder Arylalkylgruppe steht, umfasst.

9. Herstellungsverfahren nach Anspruch 8, **dadurch gekennzeichnet, dass** es einen vorab erfolgenden Schritt einer 1,4-Addition vom Michael-Typ von Benzylamin an ein ungesättigtes 1,4-Butyrolacton oder 1,4-Thiobutyrolacton umfasst, um das entsprechende 3-Benzylaminolacton zu erhalten.

10. Herstellungsverfahren nach Anspruch 9, **dadurch gekennzeichnet, dass** das Ausgangsprodukt des ersten Schritts eine D- oder L-Asparaginsäure ist und die erhaltenen Verbindungen nach den Ansprüchen 1 bis 3 optisch rein sind.

11. Herstellungsverfahren nach den Ansprüchen 7 bis 10, **dadurch gekennzeichnet, dass** die starke Base, die eingesetzt wird, um die Alkylierungen auszuführen, Lithiumhexamethyldisilazid ist.

12. Herstellungsverfahren nach den Ansprüchen 5 bis 11, **dadurch gekennzeichnet, dass** das Reagens, welches erlaubt, das Amin zu behandeln, um die Verbindungen nach den Ansprüchen 1 bis 4 zu erhalten, zu der Familie der Acylchloride, Sulfonylchloride oder Chlorformiate gehört.

13. Pharmazeutische Zusammensetzung, welche eine Verbindung der folgenden allgemeinen Formel: 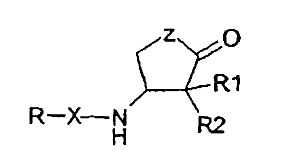 in welcher:
Z für ein Schwefel- oder Sauerstoffatom steht,
die Gruppen R1 und R2, die gleich oder voneinander verschieden sein können, jeweils für eine Alkylgruppe oder eine Alkenylgruppe stehen,
X für ein CO, ein CO₂, ein SO oder ein SO₂ steht
und die Gruppe R für eine Alkyl-, Aryl-, Alkenyl- oder Arylalkylgruppe steht,
und einen geeigneten pharmazeutischen Träger umfasst.

14. Verwendung der Verbindungen der folgenden allgemeinen Formel: 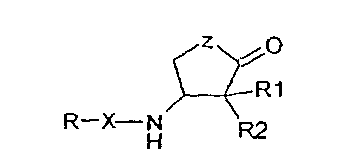 in welcher:
Z für ein Schwefel- oder Sauerstoffatom steht,
die Gruppen R1 und R2, die gleich oder voneinander verschieden sein können, jeweils für eine Alkylgruppe oder eine Alkenylgruppe stehen,
X für ein CO, ein CO₂, ein SO oder ein SO₂ steht
und die Gruppe R für eine Alkyl-, Aryl-, Alkenyl- oder Arylalkylgruppe steht, und einer Zusammensetzung nach Anspruch 13 für die Herstellung eines Stimulators der Aktivität von γ-Aminobuttersäure, welcher über den GABA-A-Rezeptor des Zentralnervensystems wirkt.

15. Verbindung der folgenden allgemeinen Formel: 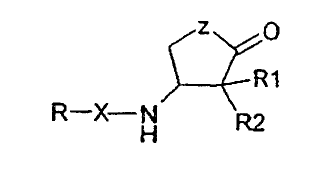 in welcher:
Z für ein Schwefel- oder Sauerstoffatom steht,
die Gruppen R1 und R2, die gleich oder voneinander verschieden sein können, jeweils für eine Alkylgruppe oder eine Alkenylgruppe stehen,
X für ein CO, ein CO₂, ein SO oder ein SO₂ steht
und die Gruppe R für eine Alkyl-, Aryl-, Alkenyl- oder Arylalkylgruppe steht,
und Zusammensetzung nach Anspruch 13 für deren Verwendung als Arzneimittel.

16. Verwendung der Verbindungen der folgenden allgemeinen Formel: 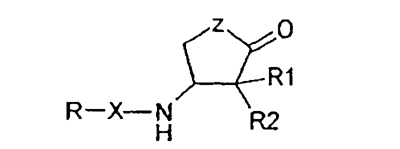 in welcher:
Z für ein Schwefel- oder Sauerstoffatom steht,
die Gruppen R1 und R2, die gleich oder voneinander verschieden sein können, jeweils für eine Alkylgruppe oder eine Alkenylgruppe stehen,
X für ein CO, ein CO₂, ein SO oder ein SO₂ steht
und die Gruppe R für eine Alkyl-, Aryl-, Alkenyl- oder Arylalkylgruppe steht,
und einer Zusammensetzung nach Anspruch 13 für die Herstellung eines Arzneimittels, welches für die Behandlung von Nervenleiden oder -störungen bestimmt ist.

17. Verwendung nach Anspruch 16, **dadurch gekennzeichnet, dass** die Nervenleiden oder -störungen vom Typ Epilepsie, Angst, Depression, Schlafstörungen, Panikattacken, Muskelkontraktionen, Schmerz, Abhängigkeit von Alkohol oder von Benzodiazepinen oder psychotisches Verhalten sind.
